# EUROPEAN PATENT SPECIFICATION

(11) **EP 1 027 888 B1**
(45) Date of publication and mention of the grant of the patent: **10.06.2009**
(21) Application number: 00300572.5
(22) Date of filing: 26.01.2000
(51) Int. Cl.: A61K 9/26

(54) **Osmotic system for delivery of solid amorphous dispersions of drugs**
Osmotisches System zur Verabreichung von Wirkstoffen, die feste amorphe Dispersionen enthalten
Dispositif osmotique pour délivrer des dispersions solides amorphes des médicaments

(30) Priority: 10.02.1999 US 119406 P
(43) Date of publication of application: 16.08.2000
(73) Proprietor: Pfizer Products Inc., Groton, CT 06340 (US)
(72) Inventor: Appel, Leah Elizabeth, Bend, Oregon 97701 (US); Curatolo, William John, Groton, Connecticut 06340 (US); Herbig, Scott Max, Groton, Connecticut 06340 (US); Nightingale, James Alan Schriver, Bend, Oregon 97708 (US); Thombre, Avinash Govind, Groton, Connecticut 06340 (US)
(74) Representative: Hill, Richard

(56) References cited:
- EP-A- 0 344 603
- EP-A- 0 901 786
- US-A- 5 035 897
- US-A- 5 516 527
- US-A- 5 736 159
- SANTUS G AND BAKER RW: "Osmotic drug delivery: a review of the patent literature" JOURNAL OF CONTROLLED RELEASE,NL,ELSEVIER SCIENCE PUBLISHERS B.V. AMSTERDAM, vol. 35, no. 1, 1 July 1995 (1995-07-01), pages 1-21, XP004037488 ISSN: 0168-3659
- YAMAGUCHI T ET AL: "Improvement of Pharmaceutical Properties of 4-O-(4- methoxyphenyl)acetyltylosin Using Solid Dispersion with Carboxymethylethylcellulose" YAKUZAIGAKU,JP,YAKUJI NIPPO-SHA, TOKYO, vol. 53, no. 4, 1993, pages 221-228, XP002121600 ISSN: 0372-7629

## Description

### BACKGROUND OF THE INVENTION

The bioavailability of sparingly water-soluble drugs is well-known to be limited and to be profoundly affected by such factors as the fed state of the patient, the rate of metabolism in relation to the rate of absorption in the gastrointestinal (GI) tract, and the dosage form. Many attempts have been made to improve the form of dosage for such low solubility drugs, generally with the aim to improving the bioavailability of such drugs. Most such formulations were immediate release in nature as this generally maximizes the amount of drug absorbed. In a few cases, sustained or delayed release dosage forms have been formulated with a view to attaining a constant rate of release of the drug in the gut over a sufficiently sustained period of time. However, most such attempts have been unsuccessful, resulting in dosage forms that generally either provide immediate release only or poor bioavailability.

Exemplary sustained release dosage forms have included an osmotic tablet comprising a semipermeable wall surrounding a compartment containing the drug and a layer of swellable hydrogel, with the crystalline drug being delivered through a passageway in the semipermeable wall by swelling of the hydrogel, as described in U.S. Patent No. 4,327,725; another osmotic tablet comprising a wall permeable to an exterior fluid but impermeable to the drug, the wall surrounding a compartment containing two osmotic agents, two expandable polymers and the drug, as described in U.S. Patent No. 4,612,008; drug dispersed in a swellable hydrogel matrix core that releases the drug by diffusion into the environment of use, as described in U.S. Patent no. 4,624,848; a hydrogel reservoir containing a multiplicity of tiny pills wherein each tiny pill consists of a wall surrounding a drug core, as described in U.S. Patent no. 4,851,232; and a two-layered tablet wherein one layer is drug mixed with a hydrogel and the other layer is a hydrogel, as described in U.S. Patent No. 5,516,527. One sustained release dosage form consists of a coated tablet with a core of a solid dispersion of drug in a swellable polyoxamer hydrogel that releases the drug by diffusion from the swollen tablet mass and by erosion of the tablet surface, as described in PCT Application No. 97/02017.

US Patent no. 5,736,159 describes a controlled release tablet comprising a core which includes a drug, an osmotic agent, a polymeric binder and a water-swellable polymer, and a membrane coating around the core.

US Patent no. 5,035,897 describes a controlled release tablet which comprises a compartment surronded by a wall. The compartment contains two layers. The first layer comprises granules of a water-soluble or unsaluble drug and a hydroxyalkylcellulose, which granules are coated with polyvinylpyrrolidone. The second layer comprises a composition that imbibes water and swells so as to expel the drug-containing granules through an exit passageway in the wall surrounding the compartment.

Solid dispersion dosage forms may be formed by solvent evaporation, by spray drying, by spraying drug solution onto the carrier in a fluidized bed granulator, by twin screw extrusion, by melt fusion, by mechanical admixture such as by ball milling and by mechanical admixture at an elevated but non-melting temperature. See, for example, PCT Application No. 93/11749; European Patent Application No. 0 552 708; U.S. Patent No. 5,456,923; Chowdary et al., 32 Indian Drugs 477 (1995); Dangprasirt et al., 21 Drug Development & Ind. Pharm. 2323 (1995); and Goracinova et al., 22 Drug Development & Ind. Pharm. 255 (1996).

However, such solid dispersion drug delivery systems have achieved very limited success in delivering poorly water-soluble drugs as they generally tend to be immediate release forms having those forms' inherent drawbacks of high peak drug concentrations in the blood, short times following administration when drug concentrations in the blood reach a maximum ("tₘₐₓ"), and relatively short duration of effective levels of concentration in the blood. In addition, although improved bioavailability relative to crystalline drug is reported, bioavailability for such dosage forms is nevertheless often low in an absolute sense. Specifically, such drug delivery systems often exhibit little overall improvement in the concentration of drug in a patient's blood over a given time period (commonly referred to as "AUC" in reference to the calculation of the area under a curve comprising a plot of concentration of drug against time).

In the case of the solid polyoxamer dispersion dosage form reported in PCT 97/02017, the dosage form suffers either from slow and incomplete release in cases when drug is released by diffusion through a membrane coating due to the inherent low solubility of the drug; conversely, where drug is released from such a dosage form by erosion of the polyoxamer, drug release is non-zero order and variable, being dependent on the patient's fed state and gastric retention time. In addition, since the polyoxamer dispersion polymers disclosed are highly hydrophilic and generally require aqueous solvents for dissolution, these polymers cannot be used to form dispersions with hydrophobic drugs via solvent processing as it is difficult or impossible to dissolve the drug and polymer in a common solvent.

There is therefore still a need in the art for a controlled release dosage form for delivery of a low solubility drug with a short elimination half-life that provides improved drug bioavailability. These needs and others which will become apparent to one skilled in the art are met by the present invention, which is summarized and described in detail below.

### BRIEF SUMMARY OF THE INVENTION

The present invention comprises a controlled release dosage form as defined in claim 1. Particular embodiments are defined in the dependent claims.

At least a major portion of the drug, *i.e*., at least 60% is amorphous (as opposed to crystalline). More preferably, substantially all of the drug, *i.e*., at least about 75%, is amorphous. Most preferably, essentially all of the drug, *i.e*., at least about 90% is amorphous. The term "drug" is conventional, denoting a compound having beneficial, prophylactic, and/or therapeutic properties when administered to an animal, especially a human.

### BRIEF DESCRIPTION OF THE SEVERAL VIEWS OF THE DRAWINGS

FIGS. 1-4 are schematics of exemplary embodiments of the invention.

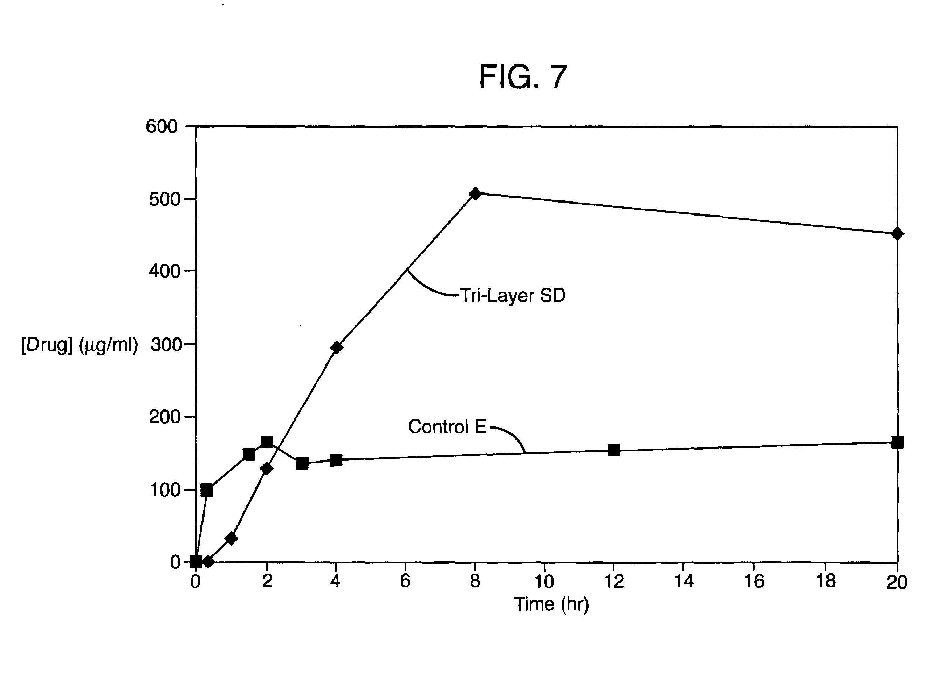

FIGS. 5-7 are graphs comprising plots of release rates of various drugs delivered by the controlled release device of the present invention and of comparative release rates for various controls.

### DETAILED DESCRIPTION OF THE INVENTION

According to the present invention there is provided a dosage form specifically designed to provide controlled release by an extrusion-type mechanism of a "low solubility" (defined below) drug that utilizes a core of such a drug in the form of a solid dispersion wherein a major portion of the drug is in an amorphous form. As used herein, the term "a major portion" of the drug means that at least 60% of the drug in the dispersion is in the amorphous form, rather than the crystalline form. Preferably, the drug in the dispersion is substantially amorphous. As used herein, "substantially amorphous" means that the amount of the drug in crystalline form does not exceed 25% as measured by powder X-ray diffraction analysis or by differential scanning calorimetry or by any other standard quantitative measurement. More preferably, the drug in the dispersion is essentially amorphous. As used herein, "essentially amorphous" means that the amount of the drug in crystalline form does not exceed 10% as measured by the methods previously described.

The term "extrusion" as it relates to the drug delivery mechanism is intended to convey an expulsion or forcing out of some or all of the core through at least one delivery port. By "at least one delivery port" is meant one or more holes, slits, passageways, channels or pores that may range in size from 0.1 to more than 2000 µm in diameter that permit release of drug from the dosage form. The drug may be delivered by the extrusion either in the form of a suspension of solids in water or primarily as a solution of the drug, to the extent dissolution has taken place in the core.

The form of the device may be any conventional form, including a tablet, a capsule, a caplet, a bead, a multiparticulate, powders for suspension or unit dosage packages or combinations thereof, and is generally useful in mammals and particularly useful for therapeutic uses in humans. Drug is released to the environment of use such as the gastro-intestinal (GI) tract as a result of the influx of water into the core and the resulting extrusion of an aqueous solution or suspension of the drug through one or more delivery ports or pores in the coating. The solid amorphous drug dispersion can either (1) dissolve in the core and be delivered primarily as a solution; or (2) be delivered as a solid suspension and dissolve in the GI tract following delivery. Since the solubility of a given drug is often pH-dependent, the device of the present invention is appropriate for delivery of any drug the solubility of which falls into the ranges noted herein.

Reference to the "release" of drug as used herein means (1) transport of drug from the interior of the dosage form to its exterior when the dosage form is a tablet, or from the interior to the exterior of the beads or granules when the dosage form includes multiparticulates, such that it contacts the fluid within a mammal's GI tract following delivery or (2) transport of drug from the interior of the dosage form such that it contacts a test medium for evaluation of the dosage form by an *in vitro* test. Reference to a "use environment" can thus be either to *in vivo* GI fluids or to an *in vitro* test medium. "Introduction" to a use environment includes either by ingestion or swallowing or use of implants or suppositories, where the use environment is *in vivo*, or being placed in a test medium where the use environment is *in vitro*.

The device comprises essentially two components: (1) a drug- and osmotic agent-containing core; and (2) a coating. The core comprises an amorphous solid dispersion of drug that contains an osmotic agent such as one or more osmogens and/or osmopolymers, and optionally contains solubility-enhancing agents and other excipients. The coating is preferably polymeric, is water-permeable, has at least one delivery port therein and does not dissolve or erode in the environment of use.

The dosage form of the present invention generally provides controlled delivery of the drug and in turn (1) the bioavailability of the drug is enhanced relative to a comparable dosage form where the drug is present in its undispersed state and (2) the time at which a maximum drug concentration (Cₘₐₓ) in an *in vitro* or *in vivo* environment of use is attained is delayed by from 30 minutes to 24 hours. By "undispersed" is meant that the drug is not formed into a solid amorphous dispersion. Rather, undispersed drug is simply the crystalline drug alone in its thermodynamically most stable form unless a crystalline form of the drug is unknown, in which case the control is the amorphous drug alone.

More specifically, the dosage forms of the invention provide one or more of the following features:
(1) they provide a Cₘₐₓ in an aqueous environment *in vitro* test, which is at least 1.2-fold that achieved by an identical controlled release dosage form containing the same quantity of undispersed drug;
(2) they provide a Cₘₐₓ in an aqueous environment *in vitro* test, at a time (tₘₐₓ) which is at least 30 minutes longer but not more than 24 hours longer than the tₘₐₓ observed when the solid dispersion is tested without incorporation into a sustained release dosage form; (3) they provide an AUC in an aqueous *in vitro* test which is at least 1.25-fold that achieved by an identical controlled release dosage form containing the same amount of undispersed drug; (4) when dosed to a human or other animal they provide a drug Cₘₐₓ in the blood that is achieved at a time tₘₐₓ which is at least 30 minutes longer than observed when a control composition is dosed, the control composition comprising the drug dispersion alone, *i.e*., not formulated in a sustained release dosage form; (5) when dosed to a human or other animal they provide a drug Cₘₐₓ in the blood that is at least 1.25-fold that observed when a control composition is dosed, the control composition being identical to the test composition with the exception that the drug is undispersed before formulation into a sustained release dosage form; and (6) when dosed to a human or other animal they provide an AUC in drug concentration in the blood that is at least 1.25-fold that observed when a control composition is dosed, the control composition being the same as that described in (5) above.

The dosage forms of the present invention can be evaluated *in vitro* by placing the dosage form into a test medium such that if all the drug dissolved, this theoretical concentration would exceed the equilibrium concentration of the undispersed drug in the same test medium by a factor of at least 2. The concentration of dissolved drug is typically measured as a function of time by sampling the drug and plotting concentration versus time so that the Cₘₐₓ can be ascertained. To avoid drug particulates which would give an erroneous determination, the test solution is either filtered or centrifuged. "Dissolved drug" is typically taken as that material that either passes a 0.45 µm syringe filter or, alternatively, the material that remains in the supernatant following centrifugation. Filtration can be conducted using a 13 mm, 0.45 µm polyvinylidine difluoride syringe filter sold by Scientific Resources under the trademark TITAN^{®}. Centrifugation is typically carried out in a polypropylene microcentrifuge tube by centrifuging at 13,000 G for 60 seconds. Other similar filtration or centrifugation methods can be employed and useful results obtained. For example, using other types of microfilters may yield values somewhat higher or lower (±10-40%) than that obtained with the filter specified above but will still allow identification of preferred dispersions. It is recognized that this definition of "dissolved drug" encompasses not only monomeric solvated drug molecules but also a wide range of species such as polymer/drug assemblies that have submicron dimensions such as drug aggregates, aggregates of mixtures of polymer and drug, micelles, polymeric micelles, colloidal particles or nanocrystals, polymer/drug complexes, and other such drug-containing species that are present in the filtrate or supernatant in the specified dissolution test.

Dosage forms of the present invention can also be tested *in vivo* in animals or humans using conventional methods for making such a determination. An *in vivo* test, such as a crossover study, may be used to determine whether a test dosage form provides an enhanced drug concentration in the blood (serum or plasma) versus time area under the curve (AUC *in vivo*) for a test subject dosed with the test dosage form relative to the AUC *in vivo* for a test subject dosed with a control dosage form as described above. In an *in vivo* crossover study a "test dosage form" is dosed to half a group of 12 or more humans and, after an appropriate washout period (*e.g*., one week) the same subjects are dosed with a "control dosage form" that comprises an equivalent quantity of undispersed drug as the "test dosage form." The other half of the group is dosed with the control dosage form first, followed by the test dosage form. The bioavailability is measured as the AUC determined for each group. AUC *in vivo* determinations can be made by plotting the blood serum or blood plasma concentration of drug along the ordinate (y-axis) versus time along the abscissa (x-axis). Generally, AUC *in vivo* values represent a number of values taken from all of the subjects in a patient test population and are, therefore, mean values averaged over the entire test population. By measuring the AUC *in vivo* for a population to which the test dosage form has been administered and comparing it with the AUC *in vivo* for the same population to which the dosage form has been administered, the test dosage form can be evaluated. The determination of AUCs is a well-known procedure and is described, for example, in the same Welling ACS Monograph mentioned above.

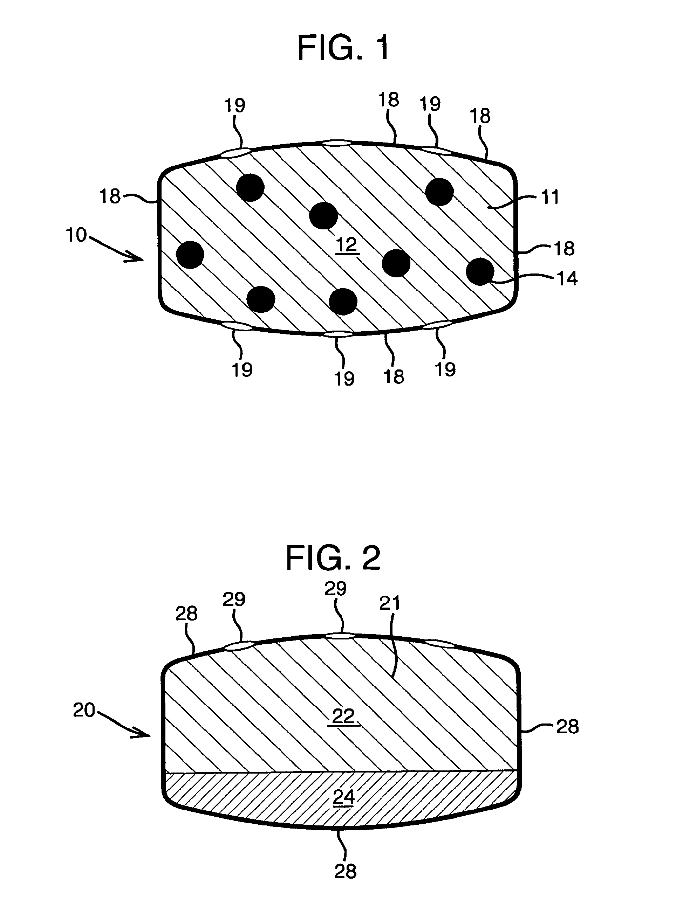

Four exemplary dosage form arrangements are schematically shown in FIGS. 1-4.
FIG. 1 depicts a "granular core" tablet 10 comprising a core 12, a coating 18 and at least one delivery port 19. The core comprises a drug-containing composition 11, and multiple granules of sweller-containing composition 14 mixed throughout the drug-containing composition 11.
FIG. 2 depicts a "bi-layer" tablet 20 comprising a core 21 that has a drug layer composition 22 and a sweller layer composition 24 and, surrounding the core, a coating 28 that has at least one delivery port 29 through the coating connecting the drug layer 22 with the exterior of the dosage form.

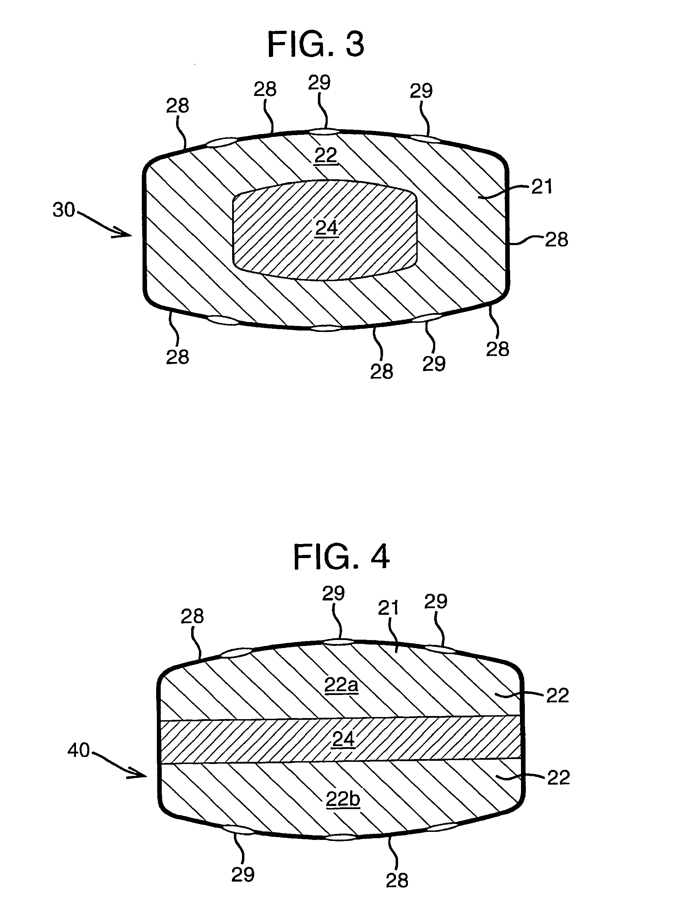

FIG. 3 depicts a "concentric core" tablet 30 comprising a core 21 that has a drug layer composition 22 that surrounds a sweller layer composition 24 and surrounding the core, a coating 28 that has at least one delivery port 29 through the coating connecting the drug layer 22 with the exterior of the dosage form.
FIG. 4 depicts a "tri-layer" tablet 40 comprising a core 21 that has two drug layer compositions 22a and 22b on either side of a sweller layer composition 24 and, surrounding the core, a coating 28 that has at least one delivery port 29 through the coating connecting each drug layer 22a and 22b with the exterior of the dosage form.

### THE DRUG

Prior to formation of the dispersion the drug in its pure state may be crystalline or amorphous, but when dispersed in the solid dispersion polymer a major portion of the drug is preferably in an amorphous or non-crystalline state, such that its non-crystalline nature is demonstrable by X-ray diffraction analysis or by differential scanning calorimetry. The dispersion may contain from about 5 to 90 wt% drug, preferably 20 to 70 wt%.

The drug is a "low-solubility drug," meaning that the drug has a minimum aqueous solubility of 40 mg/mL or less at a physiologically relevant pH (*e.g*., pH 1-8). Thus, the drug may be either substantially water-insoluble, meaning that the drug has a minimum water solubility of less than 10 micrograms/mL at a physiologically relevant pH or sparingly water-soluble, that is, has a minimum aqueous solubility of about 10 micrograms/mL up to about 1 to 2 mg/mL, or even moderate solubility, such as a minimum aqueous solubility as high as about 20 to 40 mg/mL. In general, it may be said that the drug has a dose-to-aqueous solubility ratio greater than about 5 mL, where the drug solubility is the minimum value observed in any physiologically relevant aqueous solution, including unbuffered water and USP simulated gastric and intestinal buffered solutions. In some cases, it is also desirable to enhance the solubility of the drug within the dosage form to increase the rate of release from the dosage form or to improve the absorption of drug in the colon. In such cases, the invention may be applied to drugs with solubility as high as 20 to 40 mg/mL. This is particularly true when it is desired to deliver a solution of the drug. In such cases, the dose-to-aqueous solubility ratio may be as low as 1 mL.

Virtually any beneficial therapeutic agent that meets the solubility criteria may be used as the drug in the present invention. In addition, the drug may be employed in the form of its pharmaceutically acceptable salts as well as in anhydrous, hydrated, and solvated forms and in the form of prodrugs.

Preferred classes of drugs include, but are not limited to, antihypertensives, antianxiety agents, antidepressants, barbituates, anticlotting agents, anticonvulsants, blood glucose-lowering agents, decongestants, antihistamines, antitussives, antineoplastics, beta blockers, antiinflammatories antipsychotic agents, cognitive enhancers, cholesterol-reducing agents, antiobesity agents, autoimmune disorders agents, anti-impotence agents, antibacterial and antifungal agents, hypnotic agents, anti-Parkinsonism agents, anti-Alzheimer's Disease agents, antibiotics and antiviral agents.

Specific examples of the above and other classes of drugs and therapeutic agents deliverable by the invention are set forth below, by way of example only. Specific examples of antihypertensives include prazosin, nifedipine, trimazosin and doxazosin; a specific example of a blood glucose-lowering agent is glipizide; a specific example of an anti-impotence agent is sildenafil citrate; specific examples of antineoplastics include chlorambucil, lomustine and echinomycin; a specific example of an imidazole-type antineoplastic is tubulazole; specific examples of antiinflammatory agents include betamethasone, prednisolone, aspirin, flurbiprofen and (+)-N-{4-[3-(4-fluorophenoxy)phenoxy]-2-cyclopenten-1-yl}-N-hyroxyurea; a specific example of a barbiturate is phenobarbital; specific examples of antivirals include acyclovir and virazole; specific examples of vitamins/nutritional agents include retinol and vitamin E; specific examples of a beta blockers include timolol and nadolol; a specific example of an emetic is apomorphine; specific examples of a diuretic include chlorthalidone and spironolactone; a specific example of an anticoagulant is dicumarol; specific examples of cardiotonic include digoxin and digitoxin; specific examples of androgens include 17-methyltestosterone and testosterone; a specific example of a steroidal hypnotic/anesthetic is alfaxalone; specific examples of anabolic agents include fluoxymesterone and methanstenolone; specific examples of antidepression agents include sulpiride, fluoxetine, paroxetine, venlafaxine, sertraline, [3,6-dimethyl-2-(2,4,6-trimethyl-phenoxy)-pyridin-4-yl]-(1-ethylpropyl)-amine and 3,5-dimethyl-4-(3'-pentoxy)-2-(2',4',6'-trimethylphenoxy)pyridine; specific examples of antibiotics include ampicillin and penicillin G; specific examples of anti-infectives include benzalkonium chloride and chlorhexidine; specific examples of coronary vasodilators include nitroglycerin and mioflazine; a specific example of a hypnotic is etomidate; specific examples of carbonic anhydrase inhibitors include acetazolamide and chlorzolamide; specific examples of antifungals include econazole, terconazole and griseofulvin; specific examples of anthelmintic agents include thiabendazole and oxfendazole; specific examples of antihistamines include astemizole, levocabastine, cetirizine and cinnarizine; specific examples of antipsychotics include fluspirilene, penfluridole and ziprasidone; specific examples of gastrointestinal agents include loperamide and cisapride; specific examples of serotonin antagonists include ketanserin and mianserin; a specific example of an anesthetic is lidocaine; a specific example of a hypoglycemic agent is acetohexamide; a specific example of an anti-emetic is dimenhydrinate; a specific example of an antibacterial is cotrimoxazole; a specific example of a dopaminergic agent is L-DOPA; specific examples of anti-Alzheimer's Disease agents are THA and donepezil; a specific example of an anti-ulcer agent/H2 antagonist is famotidine; specific examples of sedative/hypnotic agents include chlordiazepoxide and triazolam; a specific example of a vasodilator is alprostadil; a specific example of a platelet inhibitor is prostacyclin; specific examples of ACE inhibitor/antihypertensive agents include enalaprilic acid and lisinopril; specific examples of tetracycline antibiotics include tetracycline and minocycline; specific examples of a macrolide antibiotics include azithromycin, clarithromycin, erythromycin and spiramycin; specific examples of glycogen phosphorylase inhibitors include [R-(R*S*)]-5-chloro-N-[2-hydroxy-3-[methoxymethylamino}-3-oxo-1-(phenylmethyl)propyl]propyl]-1H-indole-2-carboxamide and 5-chloro-1H-indole-2-carboxylic acid [(1S)-benzyl-3-((3R,4S)-dihydroxypyrrolidin-1-yl-)-(2R)-hydroxy-3-oxypropyl]amide.

Still further examples of drugs deliverable by the invention are the glucose-lowering drug chlorpropamide, the anti-fungal fluconazole, the anti-hypercholesterolemic atorvastatin calcium, the antipsychotic thiothixene hydrochloride, the anxiolytics hydroxyzine hydrochloride and doxepin hydrochloride, the anti-hypertensive amlodipine besylate, the antiinflammatories iroxicam, valdecoxib and celicoxib, and the antibiotics carbenicillin indanyl sodium, becampicillin hydrochloride, troleandomycin and doxycycline hyclate.

### THE DISPERSION POLYMER

Suitable polymers for forming the solid dispersion of drug are preferably polymeric, concentration-enhancing, non-aqueous solvent-processable and inert. By "non-aqueous solvent" is meant solvents comprising <30 wt% water.

By "non-aqueous solvent-processable" is meant the polymer is capable of being processed with the drug in a common non-aqueous solvent to form the solid dispersion, *i.e*., it is generally adaptable to techniques utilizing non-aqueous solvents in the formation of solid dispersions. Such techniques include evaporation, spray-drying, or spray coating. The polymer has a preferred solubility in the non-aqueous solvent of at least 0.1 mg/mL, more preferably greater than 1 mg/mL and most preferably greater than 10 mg/mL. This property is critical for forming the solid amorphous dispersion of the drug and dispersion polymer via solvent processing as the drug and dispersion polymer must be dissolved in a common solvent and such solvents cannot be substantially aqueous as the drugs by definition have a relatively low aqueous solubility.

Such dispersion polymers are aqueous-soluble in the sense that they are sufficiently soluble (≥1 mg/mL) in at least a portion of the 1 to 8 pH range that they exhibit a "concentration enhancing" property. By "concentration-enhancing" is meant the concentration of the pure drug in aqueous media is substantially increased when formed into a solid dispersion with the polymer, the concentration enhancement being on the order of at least 20%.

By "inert" is meant not adversely reactive or bioactive, yet still capable of positively affecting the drug's bioavailability.

The amount of the polymer present in the dispersion generally ranges from about 10 to about 95 wt%, preferably 30 to 80 wt%.

The dispersion polymers used herein are selected from hydroxypropylmethylcellulose (HPMC), hydroxypropylmethylcellulose phthalate (HPMCP), hydroxypropylmethylcellulose acetate succinate (HPMCAS), cellulose acetate phthalate (CAP), cellulose acetate trimellitate (CAT), and mixtures thereof.

### THE DISPERSION

The amorphous solid dispersion of drug is prepared by solvent processing. When prepared by solvent processing, a major portion of the drug is virtually always in an amorphous state, usually substantially all (>75%) is in that state, and often essentially all (>90%) of the drug is in an amorphous state. By "amorphous state" is meant the drug may be present in the dispersion in any of three broad classes of forms: (a) in discrete, drug-rich domains; (b) homogeneously distributed therein, i.e., a solid solution; or (c) any state or combination of states between the extremes of (a) and (b).

The dispersion is formed by dissolving the drug and dispersion polymer in a common solvent, then removing the solvent by spray-drying the mixture. Spray-drying processes and equipment are described generally in Perry's Chemical Engineers' Handbook, pages 20-54 to 20-57 (6th Ed. 1984). More details on spray-drying processes and equipment are reviewed by Marshal in 50 Chem. Eng. Prog. Monogr. series 2 (1954).

The term "spray-drying" in connection with the present invention is used conventionally and broadly refers to a process involving breaking up liquid mixtures into small droplets (atomization) and rapidly removing solvent from the mixtures in a spray-drying apparatus where there is a strong driving force for evaporation of solvent from the droplets. In the case of spray-drying, the droplets generally dry prior to impinging on a surface, thus forming particles of solid amorphous dispersion on the order of 1 to 100 micrometers in diameter. The strong driving force for solvent evaporation is generally provided by maintaining the partial pressure of solvent in the spray-drying apparatus well below the vapor pressure of the solvent at the temperature of the drying droplets. This is accomplished by either (1) maintaining the pressure in the spray-drying apparatus at a partial vacuum (*e.g*., 0.01 to 0.50 atm); (2) mixing the liquid droplets with a warm drying gas; or (3) both (1) and (2). For example, a solution of drug and a dispersion polymer such as HPMCAS in acetone may be suitably spray-dried by spraying the solution at a temperature of 50°C (the vapor pressure of acetone at 50°C is about 0.8 atm) into a chamber held at 0.01 to 0.2 atm total pressure by connecting the outlet to a vacuum pump. Alternatively, such a solution may be sprayed into a chamber where it is mixed with nitrogen gas at a temperature of 80°C to 250°C and pressure of 1.0 to 1.2 atm.

Generally, the temperature and flow rate of the drying gas is chosen so that dispersion polymer/drug solution droplets are dry enough by the time they reach the wall of the apparatus that they are essentially solid, so that they form a fine powder and do not stick to the apparatus wall. The actual length of time to achieve this level of dryness depends on the size of the droplets. Droplet sizes generally are larger than about 1 µm in diameter, with 5 to 100 µm being typical. The large surface-to-volume ratio for the droplets and the large driving force for evaporation of solvent leads to actual drying times of a few seconds or less. For some mixtures of drug/dispersion polymer/solvent this rapid drying is critical to the formation of a relatively uniform, homogeneous composition as opposed to an undesirable separation into drug-rich and polymer-rich phases. Such dispersions having a homogenous composition can be considered solid solutions and may be supersaturated in drug.

Solidification times should be less than 100 seconds, preferably less than a few seconds, and more preferably less than 1 second. In general, to achieve such rapid solidification of the drug/polymer solution, it is preferred that the diameter of droplets formed during the spray-drying process are less then 100 µm, preferably less than 50 µm, and most preferably less than 25 µm. The so-formed solid particles resulting from solidification of these droplets generally tend to be 2 to 40 µm in diameter.

Following solidification, the solid powder typically remains in the spray-drying chamber for 5 to 60 seconds, evaporating more solvent. The final solvent content of the solid dispersion as it exits the dryer should be low, since low solvent content tends to reduce the mobility of drug molecules in the dispersion, thereby improving its stability. Generally, the residual solvent content of the dispersion should be less than 10 wt% and preferably less than 2 wt%.

The solution spray-dried to form the polymer/drug dispersion can be quite simple, containing only drug and polymer in a solvent. Typically, the weight ratio of polymer to drug in the solution ranges from 0.1 to 20 and preferably from 0.5 to 5. However, when the drug dose is low (less than 20 mg), the ratio may be even more than 5.

Other excipients may be added to the spray solution, either co-dissolved in the solvent along with the drug and dispersion polymer or suspended in the solution to form a slurry. Such excipients may include: acids, bases or buffers to modify the ionic state and dissolution properties of the resulting dispersion; fillers, binders, disintegrants or other materials to improve the tableting process or final properties of the tablet; antioxidants to improve the dispersion's stability; osmotic agents, including both osmotically effective solutes such as sugars, salts and polyols and water-swellable hydrophilic polymers; and surfactants to affect the wetting or dissolution rate of the tablet core.

Solvents suitable for spray-drying may be essentially any organic compound or mixtures of an organic compound and water in which the drug and polymer are mutually soluble. Because the invention utilizes low water solubility drugs, water alone is generally not a suitable solvent. However, mixtures of water and organic compounds are often suitable. Preferably, the solvent is also relatively volatile with a boiling point of 150°C or less. However, in those cases where the solubility of the drug in the volatile solvent is low, it may be desirable to include a small amount, say 2 to 25 wt%, of a low volatility solvent such as N-methylpyrrolidone (NMP), dimethylsulfoxide (DMSO) or dimethylacetamide (DMAc) in order to enhance drug solubility. Preferred solvents include alcohols such as methanol, ethanol, n-propanol, isopropanol, and butanol; ketones such as acetone, methyl ethyl ketone and methyl isobutyl ketone; esters such as ethyl acetate and propylacetate; and various other solvents such as acetonitrile, methylene chloride, toluene, and 1,1,1-trichloroethane.

### THE OSMOTIC AGENT

In addition to the solid amorphous drug dispersions, the core of the drug delivery device of the present invention includes an "osmotic agent." By "osmotic agent" is meant any agent which creates a driving force for transport of water from the environment of use into the core of the device. Exemplary osmotic agents are water-swellable hydrophilic polymers, and osmogens (or osmagens). Thus, the core may include water-swellable hydrophilic polymers, both ionic and nonionic, often referred to as "osmopolymers" and "hydrogels." The amount of water-swellable hydrophilic polymers present in the core may range from about 5 to about 80 wt%, preferably 10 to 50 wt%. Exemplary materials include hydrophilic vinyl and acrylic polymers, polysaccharides such as calcium alginate, PEO, PEG, PPG, poly(2-hydroxyethyl methacrylate), poly(acrylic) acid, poly(methacrylic) acid, PVP and crosslinked PVP, PVA, PVA/PVP copolymers and PVA/PVP copolymers with hydrophobic monomers such as methyl methacrylate, vinyl acetate, and the like, hydrophilic polyurethanes containing large PEO blocks, sodium croscarmellose, carrageenan, HEC, HPC, HPMC, CMC and CEC, sodium alginate, polycarbophil, gelatin, xanthan gum, and sodium starch glycolate. Other materials include hydrogels comprising interpenetrating networks of polymers which may be formed by addition or by condensation polymerization, the components of which may comprise hydrophilic and hydrophobic monomers such as those just mentioned. Preferred polymers for use as the water-swellable hydrophilic polymers include PEO, PEG, PVP, sodium croscarmellose, HPMC, sodium starch glycolate, polyacrylic acid and crosslinked versions or mixtures thereof. In one embodiment of the invention the osmotic agent and the dispersion polymer can comprise the same polymeric material.

By "osmotically effective solutes," is meant any water-soluble compound that is commonly referred to in the pharmaceutical arts as an "osmogen" or an "osmagent." The amount of osmogen present in the core may range from about 2 to about 70 wt%, preferably 10 to 50 wt%. Typical classes of suitable osmogens are water-soluble organic acids, salts and sugars that are capable of imbibing water to thereby effect an osmotic pressure gradient across the barrier of the surrounding coating. Typical useful osmogens include magnesium sulfate, magnesium chloride, calcium chloride, sodium chloride, lithium chloride, potassium sulfate, sodium carbonate, sodium sulfite, lithium sulfate, potassium chloride, sodium sulfate, mannitol, xylitol, urea, sorbitol, inositol, raffinose, sucrose, glucose, fructose, lactose, citric acid, succinic acid, tartaric acid, and mixtures thereof. Particularly preferred osmogens are glucose, lactose, sucrose, mannitol, xylitol and sodium chloride.

### OTHER CORE COMPONENTS

Finally, the solid dispersion core may include a wide variety of additives and excipients that enhance drug solubility or that promote stability, tableting or processing of the dispersion. Such additives and excipients include tableting aids, surfactants, water-soluble polymers, pH modifiers, fillers, binders, pigments, disintegrants, antioxidants, lubricants and flavorants. Exemplary of such components are microcrystalline cellulose; metallic salts of acids such as aluminum stearate, calcium stearate, magnesium stearate, sodium stearate, and zinc stearate; fatty acids, hydrocarbons and fatty alcohols such as stearic acid, palmitic acid, liquid paraffin, stearyl alcohol, and palmitol; fatty acid esters such as glyceryl (mono- and di-) stearates, triglycerides, glyceryl (palmiticstearic) ester, sorbitan monostearate, saccharose monostearate, saccharose monopalmitate, and sodium stearyl fumarate; alkyl sulfates such as sodium lauryl sulfate and magnesium lauryl sulfate; polymers such as polyethylene glycols, polyoxethylene glycols, and polytetrafluoroethylene; and inorganic materials such as talc and dicalcium phosphate; sugars such as lactose and xylitol; and sodium starch glycolate.

The core may also include solubility-enhancing agents that promote the water solubility of the drug, present in an amount ranging from about 5 to about 50 wt%. Examples of suitable solubility-enhancing agents include surfactants; pH control agents such as buffers, organic acids and organic acid salts and organic and inorganic bases; glycerides; partial glycerides; glyceride derivatives; polyoxyethylene and polyoxypropylene ethers and their copolymers; sorbitan esters; polyoxyethylene sorbitan esters; carbonate salts; alkyl sulfonates; and cyclodextrins.

All such solubility-enhancing and other additives may be added directly to the spray-drying solution such that the additive is dissolved or suspended in the solution as a slurry. Alternatively, such additives may be added following the spray-drying process to aid in forming the final dosage form.

### THE COATING

The essential constraints on the coating are that it be water-permeable, have at least one port for the delivery of drug, and be non-dissolving and non-eroding during release of the drug formulation, such that drug is substantially entirely delivered through the delivery port(s) or pores as opposed to delivery primarily via permeation through the coating material itself. By "delivery port" is meant any passageway, opening or pore whether made mechanically, by laser drilling, by pore formation either during the coating process or *in situ* during use or by rupture during use. Coating should be present in an amount ranging from about 5 to 30 wt%, preferably 10 to 20 wt% relative to the core weight.

A preferred form of coating is a semipermeable polymeric membrane that has the port(s) formed therein either prior to or during use. Thickness of such a polymeric membrane may vary between about 20 and 800 µm, and is preferably in the range of 100 to 500 µm. The delivery port(s) should generally range in size from 0.1 to 3000 µm or greater, preferably on the order of 50 to 3000 µm in diameter. Such port(s) may be formed post-coating by mechanical or laser drilling or may be formed *in situ* by rupture of the coatings; such rupture may be controlled by intentionally incorporating a relatively small weak portion into the coating. Delivery ports may also be formed *in situ* by erosion of a plug of water-soluble material or by rupture of a thinner portion of the coating over an indentation in the core. In addition, delivery ports may be formed during coating, as in the case of asymmetric membrane coatings of the type disclosed in U.S. Patent Nos. 5,612,059 and 5,698,220.

When the delivery port is formed *in situ* by rupture of the coating, a particularly preferred embodiment is a collection of beads that may be of essentially identical or of a variable composition. Drug is primarily released from such beads following rupture of the coating and, following rupture, such release may be gradual or relatively sudden. When the collection of beads has a variable composition, the composition may be chosen such that the beads rupture at various times following administration, resulting in the overall release of drug being sustained for a desired duration.

Coatings may be dense, microporous or "asymmetric," having a dense region supported by a thick porous region such as those disclosed in U.S. Patent Nos. 5,612,059 and 5,698,220. When the coating is dense the coating is composed of a water-permeable material. When the coating is porous, it may be composed of either a water-permeable or a water-impermeable material. When the coating is composed of a porous water-impermeable material, water permeates through the pores of the coating as either a liquid or a vapor.

Examples of osmotic devices that utilize such dense coatings include U.S. Patent Nos. 3,995,631 and 3,845,770. Such dense coatings are permeable to the external fluid such as water and may be composed of any of the materials mentioned in these patents as well as other water-permeable polymers known in the art.

The membranes may also be porous as disclosed in U.S. Patent Nos. 5,654,005 and 5,458,887 or even be formed from water-resistant polymers. U.S. Patent No. 5,120,548 describes another suitable process for forming coatings from a mixture of a water-insoluble polymer and a leachable water-soluble additive. The porous membranes may also be formed by the addition of pore-formers as disclosed in U.S. Patent No. 4,612,008.

In addition, vapor-permeable coatings may even be formed from extremely hydrophobic materials such as polyethylene or polyvinylidenefluoride that, when dense, are essentially water-impermeable, as long as such coatings are porous.

Materials useful in forming the coating include various grades of acrylics, vinyls, ethers, polyamides, polyesters and cellulosic derivatives that are water-permeable and water-insoluble at physiologically relevant pHs, or are susceptible to being rendered water-insoluble by chemical alteration such as by crosslinking.

Specific examples of suitable polymers (or crosslinked versions) useful in forming the coating include plasticized, unplasticized and reinforced cellulose acetate (CA), cellulose diacetate, cellulose triacetate, CA propionate, cellulose nitrate, cellulose acetate butyrate (CAB), CA ethyl carbamate, CAP, CA methyl carbamate, CA succinate, cellulose acetate trimellitate (CAT), CA dimethylaminoacetate, CA ethyl carbonate, CA chloroacetate, CA ethyl oxalate, CA methyl sulfonate, CA butyl sulfonate, CA p-toluene sulfonate, agar acetate, amylose triacetate, beta glucan acetate, beta glucan triacetate, acetaldehyde dimethyl acetate, triacetate of locust bean gum, hydroxlated ethylenevinylacetate, EC, PEG, PPG, PEG/PPG copolymers, PVP, HEC, HPC, CMC, CMEC, HPMC, HPMCP, HPMCAS, HPMACT, poly(acrylic) acids and esters and poly-(methacrylic) acids and esters and copolymers thereof, starch, dextran, dextrin, chitosan, collagen, gelatin, polyalkenes, polyethers, polysulfones, polyethersulfones, polystyrenes, polyvinyl halides, polyvinyl esters and ethers, natural waxes and synthetic waxes.

A preferred coating composition comprises a cellulosic polymer, in particular cellulose ethers, cellulose esters and cellulose ester-ethers, *i.e*., cellulosic derivatives having a mixture of ester and ether substituents, such as HPMCP.

Another preferred class of coating materials are poly(acrylic) acids and esters, poly(methacrylic) acids and esters, and copolymers thereof.

A more preferred coating composition comprises cellulose acetate. An even more preferred coating comprises a cellulosic polymer and PEG. A most preferred coating comprises cellulose acetate and PEG.

Coating is conducted in conventional fashion, typically by dissolving the coating material in a solvent and then coating by dipping, spray-coating or preferably by pan-coating. A preferred coating solution contains 5 to 15 wt% polymer. Typical solvents useful with the cellulosic polymers mentioned above include acetone, methyl acetate, ethyl acetate, isopropyl acetate, n-butyl acetate, methyl isobutyl ketone, methyl propyl ketone, ethylene glycol monoethyl ether, ethylene glycol monoethyl acetate, methylene dichloride, ethylene dichloride, propylene dichloride, nitroethane, nitropropane, tetrachloroethane, 1,4-dioxane, tetrahydrofuran, diglyme, and mixtures thereof. Pore-formers and non-solvents (such as water, glycerol and ethanol) or plasticizers (such as diethyl phthalate) may also be added in any amount as long as the polymer remains soluble at the spray temperature. Pore-formers and their use in fabricating coatings are described in U.S. Patent No. 5,612,059.

Coatings may also be hydrophobic microporous layers wherein the pores are substantially filled with a gas and are not wetted by the aqueous medium but are permeable to water vapor, as disclosed in U.S. Patent No. 5,798,119. Such hydrophobic but water-vapor permeable coatings are typically composed of hydrophobic polymers such as polyalkenes, polyacrylic acid derivatives, polyethers, polysulfones, polyethersulfones, polystyrenes, polyvinyl halides, polyvinyl esters and ethers, natural waxes and synthetic waxes. Especially preferred hydrophobic microporous coating materials include polystyrene, polysulfones, polyethersulfones, polyethylene, polypropylene, polyvinyl chloride, polyvinylidene fluoride and polytetrafluoroethylene. Such hydrophobic coatings can be made by known phase inversion methods using any of vapor-quench, liquid quench, thermal processes, leaching soluble material from the coating or by scintering coating particles. In thermal processes, a solution of polymer in a latent solvent is brought to liquid-liquid phase separation in a cooling step. When evaporation of the solvent is not prevented, the resulting membrane will typically be porous. Such coating processes may be conducted by the processes disclosed in U.S. Patent Nos. 4,247,498; 4,490,431 and 4,744,906.

Another embodiment of sustained release osmotic dosage forms of this invention comprises an osmotic drug-containing tablet which is surrounded by an asymmetric membrane, where said asymmetric membrane possesses one or more thin dense regions in addition to less dense porous regions. This type of membrane, similar to those used in the reverse-osmosis industry, generally allows higher osmotic fluxes of water than can be obtained with a dense membrane. When applied to a drug formulation, *e.g*. a tablet, such an asymmetric membrane allows high drug fluxes and well-controlled sustained drug release. This asymmetric membrane comprises a semipermeable polymeric material, that is, a material which is permeable to water, and substantially impermeable to salts and organic solutes such as drugs.

Materials useful for forming such an asymmetric semipermeable membrane include polyamides, polyesters and cellulose derivatives. Preferred are cellulose ethers and esters. Especially preferred are CA, CAB and EC. Especially useful materials include those which spontaneously form one or more exit passageways, either during manufacturing or when placed in an environment of use. These preferred materials comprise porous polymers, the pores of which are formed by phase inversion during manufacturing, as described above, or by dissolution of a water-soluble component present in the membrane.

The asymmetric membrane is formed by a phase-inversion process. The coating polymer, *e.g*., EC or CA, is dissolved in a mixed solvent system comprising a mixture of solvents (*e.g*., acetone) and non-solvents (*e.g*., water) for the polymer. The components of the mixed solvent are chosen such that the solvent (*e.g*. acetone) is more volatile than the non-solvent (*e.g*. water). When a tablet is contacted with such a solution and dried, the solvent component of the solvent mixture evaporates more quickly than the non-solvent. This change in solvent composition during drying causes the solution to separate into two phases so that, when solid, the polymer on the tablet is a porous solid with a thin dense outer region. This outer region possesses multiple pores through which drug can be delivered as a solution or a suspension of drug particles, which particles may be crystalline, amorphous or a drug/polymer dispersion.

In a preferred embodiment of an asymmetric membrane-coated tablet, the polymer/solvent/non-solvent mixture is sprayed onto a bed of tablets in a tablet-coating apparatus such as a Freund HCT-60 tablet coater. In this process, the tablet is coated with thick porous regions, and with a final outer thin dense region.

In the environment of use such as the GI tract, water is imbibed through the semipermeable asymmetric membrane into the tablet core. As soluble material in the tablet core dissolves, an osmotic pressure gradient across the membrane builds. When the hydrostatic pressure within the membrane enclosed core exceeds the pressure of the environment of use, the drug-containing solution is "pumped" out of the dosage form via the delivery port(s) through the semipermeable membrane. In addition the hydrostatic pressure may cause the formation of pores or even large delivery port(s) by rupture of a portion of the coating. The relatively constant osmotic pressure difference across the membrane results in a constant, well-controlled delivery of drug to the use environment. A portion of the drug dissolved in the tablet also exits via diffusion.

In this asymmetric-membrane-coated tablet embodiment, the drug may be incorporated into the dispersion in its neutral form or as a salt. It is often desirable to include one or more solubilizing excipients, such as ascorbic acid, erythorbic acid, citric acid, tartaric acid, glutamic acid, aspartic acid, glycerides, partial glycerides, glyceride derivatives, PEG, PEG esters, PPG esters, polyhydric alcohol esters, polyoxyethylene ethers, sorbitan esters, polyoxyethylene sorbitan esters, saccharide esters, phospholipids, polyethylene oxide-polypropylene oxide block co-polymers. Most preferred are the solubilizing excipients ascorbic acid, aspartic acid, citric acid, tartaric acid, glyceryl monocaprylate, glyceryl monostearate, glycerol monolaurate, and C8-C10 partial glycerides.

### USE AND FABRICATION

In use, the solid dispersion core imbibes water through the coating from the environment of use such as the GI tract so as to increase the pressure within the core. The pressure difference between the core and the device's exterior drives the delivery of the core's contents. Because the coating remains intact, the drug formulation is extruded out of the core through the delivery port(s) into the environment of use, either primarily as a solution of drug or as a suspension of drug; when delivered as a suspension, the drug formulation subsequently dissolves in the GI tract.

A preferred embodiment of osmotic delivery devices consists of a drug layer containing the substantially amorphous drug/polymer dispersion and a sweller layer that comprises a water-swellable polymer, with a coating surrounding the drug and sweller layer. Each layer may contain other excipients such as tableting aids, osmagents, surfactants, water-soluble polymers and water-swellable polymers.

Such osmotic delivery devices may be fabricated in various geometries including bilayer, wherein the core comprises a drug layer and a sweller layer adjacent to each other; trilayer, wherein the core comprises a sweller layer "sandwiched" between two drug layers; and concentric, wherein the core comprises a central sweller composition surrounded by the drug layer.

The coating of such a tablet comprises a membrane permeable to water but substantially impermeable to drug and excipients contained within. The coating contains one or more exit passageways or ports in communication with the drug-containing layer(s) for delivering the drug dispersion composition. The drug-containing layer(s) of the core contains the drug dispersion composition (including optional osmagents and hydrophilic water-soluble polymers), while the sweller layer consists of an expandable hydrogel, with or without additional osmotic agents. Such delivery devices are exemplified in FIG. 4 and Example 3 (tri-layer), FIG. 2 and Example 4 (bi-layer) and in FIG. 3 and Example 5 (concentric).

When placed in an aqueous medium, the tablet imbibes water through the membrane, causing the composition to form a dispensable aqueous composition, and causing the hydrogel layer to expand and push against the drug dispersion-containing composition, forcing the composition out of the exit passageway. The composition can swell, aiding in forcing the drug out the passageway. Drug can be delivered from this type of delivery system either dissolved or dispersed in the composition that is expelled from the exit passageway.

The rate of drug delivery is controlled by such factors as the permeability and thickness of the coating, the osmotic pressure of the drug-containing layer, the degree of hydrophilicity of the hydrogel layer, and the surface area of the device. Those skilled in the art will appreciate that increasing the thickness of the coating will reduce the release rate, while any of the following will increase the release rate: increasing the permeability of the coating; increasing the hydrophilicity of the hydrogel layer; increasing the osmotic pressure of the drug-containing layer; or increasing the device's surface area.

Exemplary materials useful in forming the drug dispersion-containing composition, in addition to the drug dispersion itself, include HPMC, PEO and PVP and other pharmaceutically acceptable carriers. In addition, osmagents such as sugars or salts, especially sucrose, lactose, xylitol, mannitol, or sodium chloride, may be added. Materials which are useful for forming the hydrogel layer include sodium CMC, PEO, poly (acrylic acid), sodium (polyacrylate), sodium croscarmelose, sodium starch glycolate, PVP, crosslinked PVP, and other high molecular weight hydrophilic materials. Particularly useful are PEO polymers having an average molecular weight from about 5,000,000 to about 7,500,000 daltons.

In general, the dosage form provides a maximum drug concentration (MDC) in a use environment that is at least 1.2-fold that of an equivalent control dosage form except that the drug is in undispersed form. When the dosage form contains homogeneous dispersions (which are preferred), the MDC value obtained when a large amount of drug is dosed can be higher for such dispersions relative to dispersions for which at least a portion of the drug is present as a drug-rich amorphous or crystalline phase.

Alternatively, the dosage form of the present invention, when tested *in vitro* in a physiologically relevant aqueous solution, for dissolution times of from 90 to 1200 minutes, provides an AUC value at least 1.25-fold that measured for an equivalent dosage form containing an equivalent quantity of undispersed drug. Preferably, when administered to a use environment, the dosage form also provides, for dissolution times of from 90 to 1200 minutes, an AUC value that is at least 1.25-fold that observed when an equivalent quantity of undispersed drug is dosed.

In the case of a bilayer geometry, the delivery port(s) or exit passageway(s) may be located on the side of the tablet containing the drug composition or may be on both sides of the tablet or even on the edge of the tablet so as to connect both the drug layer and the sweller layer with the exterior of the device. The exit passageway(s) may be produced by mechanical means or by laser drilling, or by creating a difficult-to-coat region on the tablet by use of special tooling during tablet compression or by other means. The rate of drug delivery from the device may be optimized so as to provide a method of delivering drug to a mammal for optimum therapeutic effect.

Osmotic systems can also be made with a homogeneous core surrounded by a semipermeable membrane coating. Drug dispersions can be incorporated into a tablet core that also contains other excipients that provide sufficient osmotic driving force and optionally solubilizing excipients such as acids or surfactant-type compounds. A semipermeable membrane coating can be applied via conventional tablet-coating techniques such as using a pan coater. A drug delivery passageway can then be formed in this coating by drilling a hole in the coating, either by use of a laser or other mechanical means. Alternatively, the passageway may be formed by rupturing a portion of the coating or by creating a region on the tablet that is difficult to coat, as described above.

Another embodiment of sustained release osmotic dosage forms of the invention includes drug dispersion-containing multiparticulates coated with a water-permeable membrane; the polymer may be dense, porous or asymmetric as described above. Such multiparticulates are prepared by, for example, melt-congealing from a spinning disk, extrusion/spheronization or fluid bed granulation, or by coating seed cores with a mixture of drug and a water-soluble polymer, as described above. Drug-containing multiparticulates may be homogeneous or layered with a drug dispersion surrounding the seed core. Following formation, such multiparticulates are then spray-coated with a substantially water-permeable coating comprising a solution of a polymer in a mixture of a solvent and, depending on the coating type desired, a non-solvent, as described above. This spray-coating operation is preferably carried out in a fluid bed coating apparatus, for example, a Glatt GPCG-5 fluid bed coater (Glatt Air, Ramsey, New Jersey). The polymer used for forming the semipermeable membrane is chosen as described above.

Osmotic capsules can be made using the same or similar components to those described above for osmotic tablets and multiparticulates. The capsule shell or portion of the capsule shell can be semipermeable and made of materials described above. The capsule can then be filled either by a powder or liquid consisting of drug dispersion, excipients that imbibe water to provide osmotic potential, and/or a water-swellable polymer, or optionally solubilizing excipients. The capsule core can also be made such that it has a bilayer or multilayer composition analogous to the bilayer, trilayer or concentric geometries described above.

Another class of sustained release dosage forms useful in this invention comprises coated swellable tablets and multiparticulates, as described in EP 378 404. Coated swellable tablets comprise a tablet core comprising drug dispersion and a swelling material, preferably a hydrophilic polymer, coated with a membrane which contains holes or pores through which, in the aqueous use environment, the hydrophilic polymer can extrude and carry out the drug composition. Alternatively, the membrane may contain polymeric or low molecular weight water-soluble "porosigens" which dissolve in the aqueous use environment, providing pores through which the hydrophilic polymer and drug may extrude. Examples of porosigens are water-soluble polymers such as HPMC and low molecular weight compounds such as glycerol, sucrose, glucose, and sodium chloride. In addition, pores may be formed in the coating by drilling holes in the coating using a laser or other mechanical means. In this class of sustained release dosage forms, the membrane material may comprise any film-forming polymer, including polymers which are water permeable or impermeable, providing that the membrane deposited on the tablet core is porous or contains water-soluble porosigens or possesses a macroscopic hole for water ingress and drug release. Multiparticulates (or beads) may be similarly prepared, with a drug dispersion/swellable material core, coated by a porous or porosigen-containing membrane. Embodiments of this class of sustained release dosage forms may also be multilayered, as described in EP 378 404 A2.

For any of the controlled or sustained-release dosage forms mentioned above, the dosage form may additionally comprise an immediate-release layer of the same or a different drug in crystalline, amorphous or dispersion form.

The dosage forms of the present invention are useful in treating a variety of conditions and diseases including those exemplified herein, by administering the dosage forms described herein to a patient or person in need of such treatment.

### EXAMPLE 1

Exemplary dosage forms of the present invention comprising a batch of solid dispersion ("SD") of 10 wt% sparingly soluble drug and 90 wt% polymer was made by mixing the drug [R-(R*,S*)]-5-chloro-N-[2-hydroxy-3-[methoxymethylamino)-3-1-(phenylmethyl)propyl]propyl]-1H-indole-2-carboxamide (a glycogen phosphorylase inhibitor) (hereinafter referred to as "Drug 1") having a water solubility of 1 µg/mL, in the solvent acetone together with a "medium fine" (MF) grade of HPMCAS (AQUOT, Shinetsu, Tokyo, Japan) to form a solution. The solution comprised 0.27 wt% Drug 1, 2.43 wt% polymer and 97.3 wt% solvent. This solution was then spray-dried by directing an atomizing spray via a rotary atomizer at 18 psi and a 100 g/min feed rate into a stainless steel chamber of a Niro portable spray-dryer maintained at 120°C at the inlet and 68°C at the outlet. Portions of the dispersion were held back and subjected to powder X-ray diffraction analysis and so verified the drug to be in an essentially amorphous, non-crystalline state.

The resulting solid particles had an average diameter of 5 to 20 µm. The particles were then mixed with 15 wt% of the tableting aid microcrystalline cellulose (PROSOLV, Edward Mendell Co., Patterson, New York), 30 wt% of the hydrogel PEO having an average MW OF 600,000 daltons, 4 wt% of the tableting aid HPC (KLUCEL LXF, Hercules, Wilmington, Delaware), 20 wt% of the osmotically effective solute lactose, and 1 wt% of the lubricant magnesium stearate and blended for 40 minutes to render the mixture homogeneous. The so-formed homogeneous core mixture contained 30 wt% of the solid dispersion, so that this core mixture contained 3 wt% Drug 1. This homogeneous core mixture was formed into tablet cores in a tableting press at proximately 17800N (4000 lbs) of compressive force using 10.3 mm (13/32-inch) tooling.

The tablet cores were then coated with CA (Eastman Fine Chemicals, CA 398-10, Kingsport, Tennessee) by pan-coating the same in a coating composition comprising 7 wt% CA, 3 wt% PEG having an average MW of 3350 daltons in an acetone/water mixture (68 wt%/22 wt%) and then drying the same in a convective oven at 50° C. This coating was examined by SEM and shown to contain alternating regions of (1) thick and porous, and (2) dense and thin, generally having the morphology shown in U.S. Patent No. 5,612,059. The finished weight of the core was 500 mg, while the dry coating weight was 79 mg (15.8 wt%). Twelve 0.9 mL diameter holes were then mechanically drilled in the coating of each tablet face to provide 24 delivery ports per tablet.

As a first control (Control A) a coated and drilled core was prepared that had the same composition in all respects except that no HPMCAS was present and the drug was in crystalline form. A second control (Control B), was prepared that consisted of the identical solid dispersion alone.

A Model Fasted Duodenum (MFD) solution was prepared to mimic the chemical environment of the small intestine, the solution comprising a phosphate-buffered saline solution containing 14.7 mM sodium taurocholic acid and 2.8 mM of 1-palmitoyl-2-oleyl-sn-glycero-3-phosphocholine with a pH of 6.5.

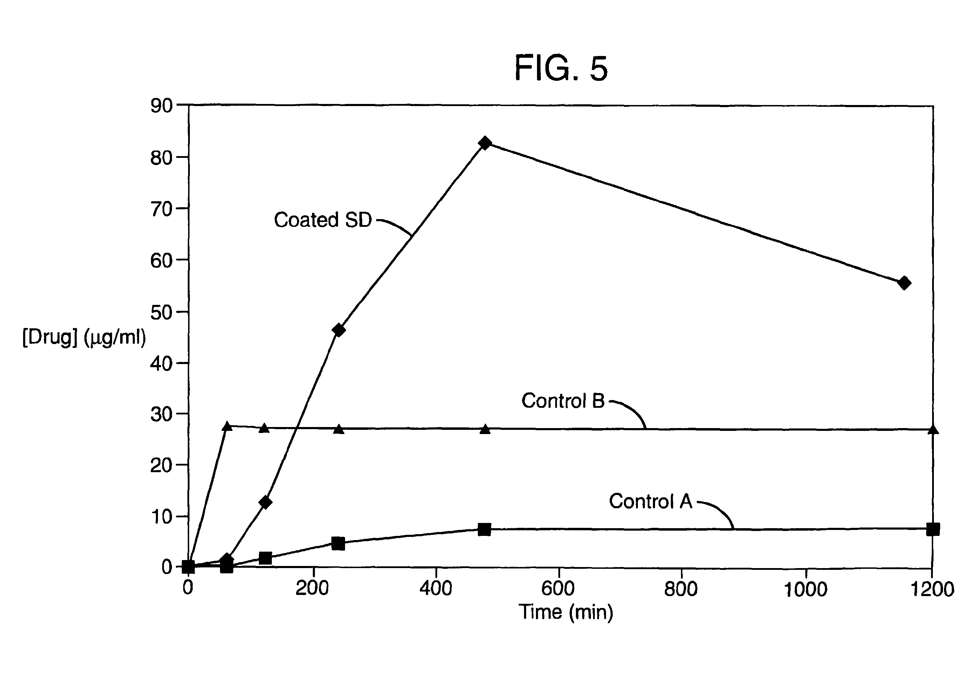

*In vitro* dissolution of drug from the three dosage forms was studied by adding each of the following dosage forms to separate 50 mL aliquots of the MFD solution at 37° C: (1) one tablet of the coated solid dispersion of the invention ("Coated SD"); (2) one tablet of Control A; and (3) 150 mg of the solid dispersion of Control B. In all three cases, the total amount of Drug 1 present was 15 mg. Thus, if all of the drug from each form dissolved, the maximum drug concentration in each test solution would be about 300 µg/mL. Drug concentration over time was determined by periodically withdrawing samples of each of the three solutions, filtering the samples to remove any undissolved drug, analyzing the samples by High Performance Liquid Chromatography (HPLC) and thereby calculating drug concentrations. The results are set forth in Table 1 and FIG. 5. As is apparent, the dosage form of the present invention exhibited dramatically higher drug concentrations over time, as well as approximately a 10-fold increase in AUC when compared to Control A.

**Table 1**

| Dosage Form Examples | Time (hrs) | [Drug] * | AUC** |
|---|---|---|---|
| Example 1 | 1 | 1.3 | 1 |
| Coated SD | 2 | 12.8 | 8 |
| | 4 | 46.5 | 67 |
| | 8 | 82.7 | 326 |
| | 19.5 | 55.9 | 1,123 |
| Control A | 1 | 0 | 0 |
| Coated | 2 | 1.8 | 1 |
| Crystalline | 4 | 4.9 | 8 |
| Drug | 8 | 7.5 | 32 |
| | 20 | 7.9 | 121 |
| Control B | 1 | 27.5 | 14 |
| Dispersion | 2 | 27.3 | 41 |
| only | 4 | 27.1 | 96 |
| | 8 | 27.2 | 206 |
| | 20 | 27.4 | 523 |

| | | | |
|---|---|---|---|
| * µg/mL **min•µg/mL | | | |

The mechanism of delivery of drug from the coated SD of the present invention (Example 1) was studied by periodic visual inspection of the tablet during drug release and revealed an imbibition of water into the core that gradually caused a swelling of the core, followed by a fairly steady extrusion of "worm"-like sections of water-swollen core material through the delivery ports, with the coating remaining intact, indicating no dissolving or eroding of the coating.

### EXAMPLE 2

A solid amorphous dispersion comprising 33.3 wt% of a different glycogen phosphorylase inhibitor, namely 5-chloro-1H-indole-2-carboxylic acid[1S)-benzyl-3-((3R,4S)-dihydroxypyrrolidin-1-yl-)-(2R)-hydroxy-3-oxypropyl]amide ("Drug 2") having a water solubility of 80 µm/mL and 66.7 wt% of the same grade of HPMCAS used in Example 1 was prepared in substantially the same way as in Example 1 except as follows: concentrations of drug and polymer in the spray solution were at 2.5 wt% and 5.0 wt%, respectively; the inlet temperature was 179°C and the outlet temperature was 70°C; the feed rate was 200 g/min and a 2-fluid nozzle was used. The solid dispersion thus formed was mixed with the other tableting excipients as in Example 1 such that the final composition was 28 wt% solid drug dispersion, 22 wt% xylitol containing 1.5 wt% CMC (XYLITAB 200, American Xyrofin, Shaumberg, Illinois), 29 wt% PEO having an average MW of 600,000 daltons, 20 wt% of the osmopolymer sodium starch glycolate (EXPLOTAB, Edward Mendell Co., Patterson, New York) and 1 wt% magnesium stearate. Tablets from this homogeneous core mixture were prepared as in Example 1, each having a core weight of 500 mg. The substantially amorphous state of the drug in the dispersion was confirmed by powder x-ray diffraction analysis.

These cores were coated as in Example 1 with CA from a coating solution comprising 7 wt% CA, 3 wt% PEG having an average MW of 3350 daltons, 5 wt% water and 85 wt% acetone, the dry coating weight amounting to 57.5 mg (11.5 wt% of tablet core). The resulting coating was dense and substantially nonporous. Five 0.9 mL diameter delivery ports were drilled in the coating as in Example 1, for a total of 10 delivery ports for each tablet.

For a comparative dissolution study, Controls C and D were prepared. Control C comprised 280 mg of the identical solid amorphous dispersion of drug with no tableting excipients and no coating, while Control D comprised a physical mixture of 93 mg of crystalline drug and 186 mg of HPMCAS-MF.

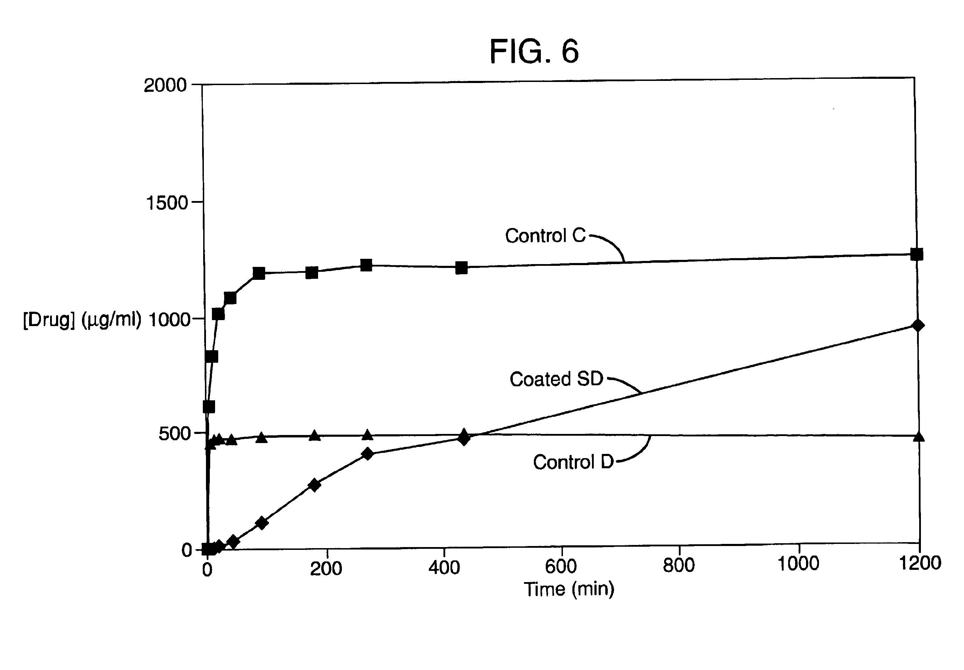

To simulate *in vivo* drug dissolution, two tablets of the invention and Controls C and D were first placed in 40 mL of a simulated gastric buffer solution for 30 minutes at 37°C and stirred, then 10 mL of MFD solution and sufficient sodium hydroxide solution to adjust the pH of the resulting solution to 6.5 were added to the gastric buffer solution. The resulting MFD solution had the same composition as that in Example 1 except that the concentrations of the taurocholic acid and the phosphocholine ester were 73.5 mM and 14 mM, respectively. Drug concentration over time was determined as in Example 1, and the results are shown in Table 2 and in the graph of FIG. 6. The data show that about 25% of the drug was released within 8 hours, and about 52% within 20 hours. This demonstrates that the dosage form of the present invention exhibits true controlled release of drug as compared to the uncoated solid drug dispersion, as the uncoated dispersion (Control C) released essentially all drug in less than an hour, generally regarded as an undesirable "spiking" of drug dosing. In addition, the concentration of drug achieved by the device of the present invention (967 µg/mL) exceeds the maximum solubility of the crystalline drug (Control D) (482 µg/mL) by a factor of two.

**Table 2**

| Dosage Form | Time (hrs) | [Drug]* | AUC** |
|---|---|---|---|
| Coated SD | 0.07 | 3.8 | 16 |
| | 0.17 | 5.1 | 42 |
| | 0.33 | 9.9 | 118 |
| | 0.67 | 27.5 | 492 |
| | 1.5 | 111 | 3,965 |
| | 3.0 | 275 | 21,370 |
| | 4.5 | 405 | 59,990 |
| | 7.25 | 473 | 124,500 |
| | 20 | 967 | 675,000 |
| Control C | 0.07 | 613 | 1,090 |
| Uncoated SD | 0.17 | 830 | 4,540 |
| | 0.33 | 1,020 | 17,760 |
| | 0.67 | 1,090 | 32,560 |
| | 1.5 | 1,200 | 87,890 |
| | 3.0 | 1,200 | 193,300 |
| | 4.5 | 1,230 | 301,300 |
| | 7.25 | 1,215 | 501,000 |
| | 20 | 1,270 | 1,442,000 |
| Control D | 0.07 | 456 | 1,160 |
| Crystalline | 0.17 | 470 | 3,930 |
| Drug mixed | 0.33 | 475 | 8,660 |
| with | 0.67 | 471 | 18,110 |
| dispersion | 1.5 | 480 | 41,840 |
| polymer | 3.0 | 483 | 85,210 |
| | 4.5 | 488 | 128,900 |
| | 7.25 | 493 | 209,800 |
| | 20 | 482 | 582,600 |

| | | | |
|---|---|---|---|
| * µg/mL **min•µg/mL | | | |

The mechanism of release from the coated solid dispersion of the invention was studied as in Example 1, and confirmed that the coating neither dissolved nor eroded, while the solid dispersion in the core was extruded as a solid dispersion which thereafter dissolved in the combined gastric/MFD solution.

### EXAMPLE 3

This example illustrates the inventive delivery of Drug 2 except that the core of the tablet was a "trilayer" design of the type depicted in FIG. 4. The core comprised two compositions; a sweller layer between two essentially identical drug layers. The drug was in the form of a solid dispersion of identical composition to that of Example 2. The drug layers were composed of 26.25 wt% solid dispersion, 35 wt% XYLITAB 200, 32.5 wt% PEO with an average MW of 600,000 daltons, 5.0 wt% EXPLOTAB and 1.25 wt% magnesium stearate. The sweller layer was composed of 74 wt% EXPLOTAB, 25 wt% PROSOLV 90 and 1 wt% magnesium stearate. Tablets were formed by first mechanically mixing the above drug layer ingredients until homogeneous, compressing to a hardness of 39 N (4 Kponds) and then milling to a size of 800 µm or less. 200 mg of the drug layer mixture were then placed into the bottom of a standard 9.5 mm (3/8-inch) die and lightly tamped. Then 100 mg of the sweller layer mixture was placed in the die on top of the drug layer and lightly tamped. Finally, an additional 200 mg of the drug layer mixture was placed in the die on top of the sweller layer. The tablet was then compressed to a hardness of 98 to 137 N. (10 to 14 Kponds) with the tooling described in Example 1.

The resulting trilayer core thus had a total weight of 500 mg and contained 105 mg of solid dispersion, 35 mg of which was Drug 2. The trilayer core was then coated and ports drilled as in Example 2 except that the coating weight was 11.8 wt% of the core weight.

For a comparative dissolution study, Control E, consisting of 35 mg of crystalline Drug 2 was prepared. The dissolution of drug was studied by placing two of the trilayer tablets and Control E each in 35 ml of a phosphate buffered saline (PBS) solution composed of 6 mM KH₂PO₄, 5 mM NaOH, 60 mM KC1 and 30 mM NaCl. Drug concentration over time was determined as in Example 1 and the results are shown in Table 3. The data for the trilayer tablet is the average of the values for the two tablets tested.

The data shows that drug was gradually released from the trilayer tablet, reaching a maximum concentration after about eight hours, and demonstrating sustained release of drug. In addition, the MDC achieved by the trilayer tablet was 510 µg/mL, nearly three-fold the 171 µg/mL observed for pure crystalline drug.

**Table 3**

| Dosage Form | Time (hrs) | [Drug] * |
|---|---|---|
| Trilayer SD | 0.3 | - |
| | 1.0 | 32 |
| | 1.5 | - |
| | 2.0 | 129 |
| | 3.0 | - |
| | 4.0 | 297 |
| | 8.0 | 510 |
| | 12.0 | - |
| | 20.0 | 459 |
| Control E | 0.3 | 99 |
| Crystalline | 1.0 | - |
| Drug | 1.5 | 148 |
| | 2.0 | 165 |
| | 3.0 | 137 |
| | 4.0 | 142 |
| | 8.0 | - |
| | 12.0 | 157 |
| | 20.0 | 171 |

| | | |
|---|---|---|
| *µg/mL | | |

### EXAMPLE 4

This example illustrates a method for making a dosage form of the present invention in a bilayer core geometry of the type depicted in FIG. 2. The bilayer core consists of a drug layer and a sweller layer. To form the drug layer, the following materials may be blended and wet-granulated in a mixer: 50 g to 200 g of the drug dispersion of Example 2; 250 g to 325 g of a PEO having an average MW of about 200,000 daltons; 10 to 30 g of an HPMC having an average MW of about 11,300; and 0 to 10 g of magnesium stearate. The sweller layer may be formed by wet-granulating the following materials: 110 g to 140 g of PEO having an average MW of about 5,000,000 to 7,500,000 daltons; 5 to 25 g of an HPMC having a MW of about 11,300 daltons; 40 g to 70 g sucrose; and 0 to 10 g magnesium stearate. The bilayer core is formed by first placing 50 mg to 300 mg of the sweller layer granulation into the bottom of a 9.5 mm (3/8-inch) die and then lightly tamping the material. On top of this sweller layer is then placed 50 mg to 300 mg of the drug layer granulation. The tablet is then compressed to a hardness of 59 to 147N. (6 to 15 Kponds). The resulting bilayer cores are then coated with a semipermeable coating comprising 50% to 98% CA having an acetyl content of about 32 to 40 wt% and from 2 to 50% PEG having an average MW of about 3,350 daltons. In the coating, at least one exit passageway from 500 to 2,000 µm in diameter is formed on the drug layer face of the tablet.

### EXAMPLE 5

This example illustrates the fabrication of the inventive dosage form except that the core of the device is of a concentric design of the type depicted in FIG. 3. The core consists of a sweller composition central core surrounded by the drug composition. To form the central core, 100 to 200 mg of the wet-granulated sweller mixture of Example 3 is placed in the bottom of a 6.4 mm (1/4-inch) die and compressed to a hardness of about 39 to 59N (4 to 6 Kponds). 200 to 300 mg of the drug-containing composition of Example 3 is then placed in the bottom of a 10.3 mm (13/32-inch) die and leveled by hand using a spatula. The previously formed central core is then placed on top of the layer of drug-containing composition at its center and then an additional 200 to 300 mg of the same composition is placed on top of and around the central core. The material is then compressed to a hardness of 98 to 137N (10 to 14 Kponds) to form a concentric design tablet core, which is then coated with a semipermeable coating as described in Example 3. Finally, at least one exit passageway from 500 to 2,000 µm in diameter is formed on each face of the coated tablet.

### EXAMPLE 6

This example illustrates the delivery of an SD of Drug 1 and HPMCAS-MF from a tablet wherein the core is of the tri-layer design depicted in FIG. 4. The core was made up of two compositions: a sweller layer between two essentially identical drug layers, each drug layer comprising an SD prepared as in Example 1, except that the SD contained 67 wt% Drug 1 and 33 wt% HPMCAS-MF.

The drug layers comprised 9.56 wt% SD, 38.4 wt% XYLITAB 200, 37.3 wt% PEO with an average MW of 600,000 daltons, 13.5 wt% EXPLOTAB, and 1.24 wt% magnesium stearate. The sweller layer was 74.5 wt% EXPLOTAB, 25 wt% microcrystalline cellulose (AVICEL PH 200, FMC Corporation, Philadelphia, Pennsylvania) and 0.5 wt% magnesium stearate. Tablets were formed by mechanically mixing the drug layer ingredients until homogeneous. The sweller layer ingredients were then mixed until homogeneous. Tablets were formed by first placing 200 mg of the drug layer mixture in a standard 10.3 mm (13/32-inch) die and tamping lightly. Then, 100 mg of the sweller layer mixture were placed in the same die on top of the first drug layer, followed by 200 mg of a second drug layer mixture, and compressing to a hardness of 98N (10 Kp).

The resulting tri-layer core thus had a total weight of 500 mg and contained 38.25 mg of SD. The SD was determined to have a Drug 1 activity and potency of 65.4% and 90.5%, respectively. Thus, the tri-layer core contained 122.6 mg of Drug 1. The tri-layer core was then coated and ports drilled as in Example 2, except that the coating weight was 12.9% of the core weight.

For a comparative dissolution study, Control F, consisting of 22.64 mg of crystalline Drug 1, was prepared. The dissolution of drug was studied by placing two each of the tri-layer tablets and Control F preparations containing an equal amount of Drug 1 in 50 ml of the MFD solution of Example 1. Drug concentration over time was determined as in Example 1 and the results are shown in Table 4. The data for the tri-layer tablet is the average of the values for the two tablets tested.

The data show that the drug was gradually released from the tri-layer tablet, reaching a maximum concentration after about 8 hours. In addition, the MDC achieved by the tri-layer tablet was 151 µg/mL, nearly 10 times higher than the 16 µg/mL observed for the pure crystalline drug.

**Table 4**

| Dosage Form | Time (hr) | [Drug]* |
|---|---|---|
| Tri-layer | 0 | 0 |
| Coated SD | 2 | 51 |
| (average of 2) | 4 | 130 |
| | 8 | 151 |
| | 12 | 101 |
| | 20 | 57 |
| | 24 | 39 |
| Control F | 0 | 0 |
| Crystalline | 2 | 13 |
| Drug | 4 | 16 |
| | 8 | 13 |
| | 12 | 15 |
| | 20 | 12 |
| | 24 | 11 |

| | | |
|---|---|---|
| *µg/mL | | |

### EXAMPLE 7

This example illustrates the delivery of Drug 2 from a tablet wherein the core is of the bi-layer design shown in FIG. 2. The core comprised a sweller layer and a drug layer. The drug layer was in the form of an SD comprising 50 wt% of Drug 2 having a water solubility of 80 µg/mL and 50% HPMCAS-MF. The SD was prepared in essentially the same way as Example 1 except that the spray solution comprised 7.5 wt% Drug 2, 7.5 wt% polymer and 85 wt% 95:5 acetone:H₂O. This solution was spray-dried using an external mix 2-fluid atomizer with an atomizing gas feed rate of 460 g/min and a 200 g/min solution feed rate with an inlet temperature of 195°C and an outlet temperature of 70°C.

The resulting solid particles had an average diameter of approximately 50 µm. The drug layer comprised 44.4 wt% SD, 26.1 wt% XYLITAB 200, 25.2 wt% PEO with an average MW of 600,000 daltons, 3.5 wt% EXPLOTAB, and 0.8 wt% magnesium stearate. The sweller layer was 74.5 wt% EXPLOTAB, 25 wt% PROSOLV 90, and 0.5 wt% magnesium stearate. Tablets were formed by first mechanically mixing the drug layer ingredients until homogeneous, compressing the same into lightly compacted tablets, and grinding the resulting tablets to particles less than 16 mesh in size. The sweller layer ingredients were then mixed until homogeneous. Tablets were formed by first placing 450 mg of drug layer mixture in a standard 12 mm (15/32-inch) die and tamping lightly, then placing 150 mg of the sweller layer mixture in the die on top of the drug layer and compressing to a hardness of 147N (15 Kp).

The resulting bi-layer core thus had a total weight of 600 mg and contained 199.8 mg SD, 99.9 mg of which was Drug 2. This core was then coated and ports drilled as in Example 2, except that the coating weight was 8.9% of the core weight, and five 900 µm holes were drilled on the drug layer side only.

For a comparative dissolution study, Control G, consisting of 100 mg of crystalline Drug 2 was prepared. The dissolution of drug was studied by placing one each of the bi-layer tablet and Control G preparation containing an equal amount of Drug 2 in 50 ml of the phosphate-buffered solution of Example 3 (pH 7.2, osmotic pressure 5.3 atm) to simulate an intestinal environment of use. Drug concentration over time was determined as in Example 1, and the results are shown in Table 5.

The data show that the drug was gradually released from the bi-layer tablet, reaching an MDC after about 8 hours, and demonstrating sustained release of the drug. In addition, the MDC achieved by the bi-layer tablet was 608 µg/mL, nearly eight times higher than the 77 µg/mL observed for pure crystalline drug.

**Table 5**

| Dosage Form | Time (hr) | [Drug]* |
|---|---|---|
| Bi-layer | 0 | 0 |
| Coated SD | 1 | 4 |
| | 2 | 66 |
| | 4 | 350 |
| | 8 | 608 |
| | 12 | 306 |
| | 18 | 263 |
| | 24 | 298 |
| Control G | 0 | 0 |
| Crystalline | 1 | 59 |
| Drug | 2 | 72 |
| | 4 | 72 |
| | 8 | 77 |
| | 12 | 71 |
| | 18 | 71 |
| | 24 | 75 |

| | | |
|---|---|---|
| *µg/mL | | |

### EXAMPLE 8

Exemplary dosage forms of the present invention for sertraline (a low solubility serotonin reuptake inhibiting drug) were made with a bi-layer core geometry of the type depicted in FIG. 2. The bi-layer core consisted of a layer of a sertraline-containing composition and a layer of a water-swellable composition. The sertraline-containing composition was in the form of a solid dispersion comprising 50 wt% of the drug and 50 wt% HPMCP-55. The solid dispersion was prepared in essentially the same way as in Example 1 except as follows: the solution comprised 2.5 wt% drug, 2.5 wt% polymer, 47.5 wt% methanol, and 47.5 wt% acetone. This solution was spray-dried using a 2-fluid nozzle with an atomization pressure of 1.8 bar and a 193 g/min feed rate. The inlet temperature was maintained at 230°C and the outlet temperature was 72°C.

The drug layer was composed of 41.15 wt% solid dispersion, 26.75 wt% XYLITAB 200, 26.75 wt% PEO with an average MW of 600,000 daltons, 4.33 wt% EXPLOTAB, and 1.02 wt% magnesium stearate. The sweller layer was composed of 74.66 wt% EXPLOTAB, 24.73 wt% PROSOLV 90, 0.47 wt% magnesium stearate, and 0.14 wt% Red Lake #40. Tablets were formed by first combining the above drug layer ingredients, precompressing, and milling in a comill at 1100 rpm (screen size 1.9 mm (0.075 inch)). The sweller layer ingredients were combined without the magnesium stearate, blended 20 minutes in a Turbula mixer, then blended again for 4 minutes with magnesium stearate. Each tablet was made using 550 mg drug layer and 150 mg sweller layer, and compressed to a hardness of 113N (11.5 Kp).

The resulting bilayer core thus had a total weight of 700 mg and contained 226.3 mg of solid dispersion, 113.2 of which was sertraline. The bilayer core was then coated and ports drilled as previously described, except that the coating weight was 9.3% of the core weight, and one 700 µm hole was drilled.

For a comparative dissolution study, Control H, consisting of 111.4 mg of the crystalline drug, was used. The dissolution of drug was studied by placing the bilayer tablet and Control H each in 40 ml of MFD solution. Drug concentration over time was determined as in Example 1, and the results are shown in Table 6.

**Table 6**

| Dosage Form | Time (hr) | [Drug] * | AUC |
|---|---|---|---|
| Bilayer SDD | 0 | 0 | 0 |
| | 0.2 | 0 | 0 |
| | 0.3 | 0 | 0 |
| | 0.7 | 0 | 0 |
| | 1.5 | 32 | 800 |
| | 2 | 83 | 2,530 |
| | 3 | 307 | 14,250 |
| | 4 | 465 | 37,410 |
| | 6 | 566 | 99,260 |
| | 10 | 262 | 198,640 |
| | 14 | 176 | 224,940 |
| | 24 | 297 | |
| Control H | 0 | 0 | 0 |
| Crystalline | 0.2 | 278 | 830 |
| Drug | 0.3 | 401 | 4,230 |
| | 0.7 | 341 | 11,650 |
| | 1.5 | 217 | 25,610 |
| | 2 | 188 | 31,700 |
| | 3 | 168 | 42,400 |
| | 4 | 152 | 52,000 |
| | 6 | 160 | 70,640 |
| | 10 | 137 | 106,200 |
| | 14 | 135 | 122,500 |
| | 24 | 191 | |

| | | | |
|---|---|---|---|
| * µg/mL ** min·µg/mL | | | |

The data shows that the drug was gradually released from the bilayer tablet, reaching a maximum concentration after about 6 hours and demonstrating sustained release of the drug. The MDC achieved by the bilayer tablet was 566 µg/mL, higher than the 401 µg/mL observed for pure crystalline drug. At 14 hours, the AUC for the bilayer tablet was 1.8 times the AUC for the control.

### EXAMPLE 9

Multiparticulates that provide controlled-release of a drug formed as a solid amorphous dispersion can be made by making drug-containing cores via a melt-congealing process and then applying a water-permeable coating around these cores. To make the drug-containing cores, a drug dispersion made as in Example 1 is mixed with glyceryl behenate (COMPRITOL 888 ATO, Gattefosse Corporation, Westwood, New Jersey) and PEO with an average MW of 600,000 daltons. This mixture consists of 50 wt% COMPRITOL, 25 wt% PEO, and 25 wt% drug dispersion and is mixed in a stirred and heated vessel. The mixture is heated to 85°C, melting the COMPRITOL and forming a suspension of PEO and drug dispersion. This suspension is pumped to a rotary atomizer to form droplets that solidify upon cooling to form drug dispersion-containing beads. The suspension is pumped to the rotating disc of the atomizer at a rate of about 1 kg/hr and at a rotary speed of approximately 3600 rpm. The solidified beads are collected and sieved to remove fines and any large particles or clumps.

A water-permeable coating is then applied to these beads via a conventional fluid bed coating process. A coating solution consisting of 7 wt% CA 389-10, PEG 3350, and 18 wt% water dissolved in acetone is prepared in a stirred vessel. The coating solution is spray-coated onto the beads via a bottom-spray fluid bed coater fitted with a Wurster insert. Coating solution is applied until a coating weight of approximately 15 wt% (relative to core weight) is achieved. Drug delivery ports are formed in the environment of use by imbibition of water into the bead cores, causing the core material to swell and rupture the coating. The core material is then extruded out through the rupture in the coating, providing controlled release of the drug dispersion.

### EXAMPLE 10

Multiparticulates that provide controlled release of a drug formed as a solid amorphous dispersion can be made by making drug-containing cores via a spray-coating process and then applying a water-permeable coating around these cores. To make the drug-containing cores a drug solution is spray-coated onto seed cores via a conventional fluid bed coating process. The drug solution consists of 1 wt% drug, 1 wt% PEO with an average MW of 600,000 daltons, and 3 wt% HPMCAS dissolved in acetone. This drug solution is coated onto microcrystalline cellulose spheres (CELPHERE CP-507, FMC Corporation, Philadelphia, Pennsylvania) having a nominal diameter of 600 µm in a fluid bed coater fitted with a Wurster insert. Coating is applied to the cores until a coating weight of approximately 100 wt% compared to the original weight of the cores is achieved. An amorphous drug dispersion is formed via this spray-coating process that also forms drug-containing beads.

A water-permeable coating is then applied to these beads via a conventional fluid bed coating process. A coating solution consisting of 7 wt% CA 389-10, PEG 3350, and 18 wt% water dissolved in acetone is prepared in a stirred vessel. The coating solution is spray-coated onto the beads via a bottom-spray fluid bed coater fitted with a Wurster insert. Coating solution is applied until a coating weight of approximately 15 wt% compared to the original weight of the beads is achieved. Drug delivery ports are formed in these coatings in the environment of use by imbibition of water into the bead cores, causing the core material to swell and rupture the coating. The core material is then extruded out through the rupture in the coating, providing controlled release of the drug dispersion.

## Claims

1. A controlled release dosage form comprising:
(a) a core comprising an osmotic agent and a drug in the form of a spray-dried solid dispersion of said drug in a dispersion polymer selected from hydroxypropylmethylcellulose, hydroxypropylmethylcellulose phthalate, hydroxypropylmethylcellulose acetate succinate, cellulose acetate phthalate, cellulose acetate trimellitate and mixtures thereof, wherein the drug has an aqueous solubility of 40mg/mL or less at a physiologically relevant pH, at least 60% of said drug being amorphous; and
(b) a substantially water-permeable coating around said core having at least one delivery port therein, said coating controlling the influx of water to said core from an aqueous environment of use so as to cause extrusion of at least a portion of said core through said at least one delivery port to said aqueous environment of use, said coating being non-dissolving and non-eroding during release of said drug.

2. The dosage form of claim 1 wherein at least 75% of said drug is amorphous.

3. The dosage form of claim 1 wherein at least 90% of said drug is amorphous.

4. The dosage form of claim 1 wherein said coating is a polymeric membrane.

5. The dosage form of claim 4 wherein said polymeric membrane is semipermeable.

6. The dosage form of claim 4 wherein said polymeric membrane is porous.

7. The dosage form of claim 4 wherein said polymeric membrane comprises at least one asymmetric membrane.

8. The dosage form of claim 7 wherein said at least one delivery port comprises pores in said coating.

9. The dosage form of claim 1 wherein said at least one delivery port is formed by laser drilling.

10. The dosage form of claim 1 wherein said at least one delivery port is formed in said environment of use.

11. The dosage form of claim 10 wherein said at least one delivery port is formed by the erosion of a plug of water-soluble material.

12. The dosage form of claim 10 wherein said coating is rupturable to form said at least one delivery port.

13. The dosage form of claim 12 wherein said at least one delivery port is formed by a rupture of a relatively small portion of said coating.

14. The dosage form of claim 13 wherein said rupture takes place in a thinner portion of said coating over an indentation in said core.

15. The dosage form of claim 4 wherein said coating is formed from a polymer selected from the group consisting of poly(acrylic) acids and esters; poly(methacrylic) acids and esters; copolymers of poly(acrylic) and poly(methacrylic) acids and esters; cellulose esters; cellulose ethers; and cellulose ester/ethers.

16. The dosage form of claim 4 wherein said coating is formed from a polymer selected from the group consisting of polyethylene glycol, polypropylene glycol, copolymers of polyethylene glycol and polypropylene glycol, poly(vinylpyrrolidone), ethyl cellulose, hydroxyethyl cellulose, hydroxypropyl cellulose, carboxymethyl cellulose, carboxymethylethyl cellulose, starch, dextran, dextrin, chitosan, collagen, gelatin, bromelain, cellulose acetate, unplasticized cellulose acetate, plasticized cellulose acetate, reinforced cellulose acetate, cellulose acetate phthalate, cellulose acetate trimellitate, hydroxypropylmethylcellulose, hydroxypropylmethyl-cellulose phthalate, hydroxypropylmethylcellulose acetate succinate, hydroxypropylmethylcellulose acetate trimellitate, cellulose nitrate, cellulose diacetate, cellulose triacetate, agar acetate, amylose triacetate, beta glucan acetate, beta glucan triacetate, acetaldehyde dimethyl acetate, cellulose acetate ethyl carbamate, cellulose acetate phthalate, cellulose acetate methyl carbamate, cellulose acetate succinate, cellulose acetate dimethaminoacetate, cellulose acetate ethyl carbonate, cellulose acetate chloroacetate, cellulose acetate ethyl oxalate, cellulose acetate methyl sulfonate, cellulose acetate butyl sulfonate, cellulose acetate propionate, cellulose acetate p-toluene sulfonate, triacetate of locust gum bean, cellulose acetate with acetylated hydroxyethyl cellulose, hydroxlated ethylenevinylacetate, cellulose acetate butyrate, polyalkenes, polyethers, polysulfones, polyethersulfones, polystyrenes, polyvinyl halides, polyvinyl esters and ethers, natural waxes and synthetic waxes.

17. The dosage form of claim 1 in a form selected from the group consisting of a tablet, a capsule a bead and a collection of at least two types of beads having different drug release properties, and wherein the environment of use is a human gastrointestinal tract.

18. The dosage form of claim 1 wherein said osmotic agent is selected from the group consisting of an osmotically effective solute and a water-swellable hydrophilic polymer

19. The dosage form of claim 18 wherein said osmotic agent is water-swellable and substantially segregated from said solid dispersion.

20. The dosage form of claim 19 wherein said osmotic agent and said solid dispersion are in respective discrete layers.

21. The dosage form of claim 20 wherein said osmotic agent is in a first layer and said solid dispersion is in a second layer.

22. The dosage form of claim 21, including solid dispersion in a third layer wherein said osmotic agent is between said first layer and said second layer.

23. The dosage form of claim 19 wherein said solid dispersion surrounds said osmotic agent.

24. The dosage form of claim 1 wherein said osmotic agent and said dispersion polymer are the same.

25. The dosage form of claim 18 wherein said water-swellable hydrophilic polymer is selected from the group consisting of hydrophilic vinyl and acrylic polymers, polysaccharide alginates, poly(ethylene oxide), polyethylene glycol, polypropylene glycol, poly(2-hydroxyethyl methacrylate), poly(acrylic) acid, poly(methacrylic) acid, polyvinyl pyrrolidone, crosslinked polyvinyl pyrrolidone, polyvinyl alcohol, polyvinyl pyrrolidone/polyvinyl alcohol copolymers, vinyl acetate, hydrophilic polyurethanes containing large polyethylene oxide blocks, carrageenan, hydroxethylcellulose, hydroxypropylcellulose, hydroxypropylmethylcellulose, carboxymethylcellulose, carboxyethylcellulose, sodium alginate, polycarbophil, gelatin, xanthan gum, sodium croscarmellose, and sodium starch glycolate.

26. The dosage form of claim 25 wherein said water-swellable hydrophilic polymer is selected from the group consisting of polyethylene oxide, polyethylene glycol, carboxymethylcellulose, polyvinyl pyrrolidone, hydroxypropylmethylcellulose, poly(acrylic) acid, cross-linked poly(acrylic) acid, sodium croscarmellose and sodium starch glycolate.

27. The dosage form of claim 1 wherein said core further comprises a solubility-enhancing agent.

28. The dosage form of claim 27 wherein said solubility-enhancing agent is selected from the group consisting of organic acids and organic acid salts; partial glycerides; glycerides; glyceride derivatives; polyethylene glycol esters; polypropylene glycol esters; polyhydric alcohol esters; polyoxyethylene ethers; sorbitan esters; polyoxyethylene sorbitan esters; carbonate salts; and cyclodextrins.

29. The dosage form of claim 1 wherein, prior to formation of said solid dispersion, said drug is amorphous.

30. The dosage form of claim 1 wherein, prior to formation of said solid dispersion, said drug is crystalline.

31. The dosage form of claim 1 wherein said core further comprises excipients.

32. The dosage form of claim 31 wherein said excipients are selected from the group consisting of surfactants, water-soluble polymers, pH modifiers, fillers, binders, pigments, lubricants, antioxidants and flavorants.

33. The dosage form of claim 1 wherein said drug is selected from the group consisting of an anti-hypertensive, and antianxiety agent, an anticlotting agent, a blood glucose-lowering agent, a decongestant, an antihistamine, an antitussive, an anti-inflammatory, an anti-atherosclerotic agent, an antipsychotic agent, a cognitive enhancer, a cholesterol-reducing agent, an antiobesity agent, an autoimmune disorders agent, a hypnotic agent, an anti-Parkinsonism agent, an antibiotic, an antiviral agent, an anti-impotence agent, an anti-neoplastic, a sedative, a barbituate, a nutritional agent, a beta-blocker, an emetic, an anti-emetic, a diuretic, an anticoagulant, a cardiotonic, an androgen, a corticoid, an anabolic agent, an anti-depression agent, an anti-infective agent, a coronary vasodilator, a carbonic anhydrase inhibitor, an antifungal, an antiprotozoal, a gastrointestinal agent, a dopaminergic agent, an anti-Alzheimer's Disease agent, an anti-ulcer agent, a platelet inhibitor, and a glycogen phosphorylase inhibitor.

34. The dosage form of claim 33 wherein said drug is an antihypertensive selected from the group consisting of prazosin, nifedipine, trimazosin and doxazosin.

35. The dosage form of claim 33 wherein said drug is the antipsychotic agent ziprasidone.

36. The dosage form of claim 33 wherein said drug is the blood glucose-lowering agent glipizide.

37. The dosage form of claim 33 wherein said drug is an anti-impotence agent selected from the group consisting of sildenafil and pharmaceutically acceptable salts thereof.

38. The dosage form of claim 33 wherein said drug is the anti-inflammatory agent (+)-N-{4-[3-(4-fluorophenoxy)phenoxy]-2-cyclopenten-1-yl}-N-hyroxyurea.

39. The dosage form of claim 33 wherein said drug is an antidepression agent selected from the group consisting of fluoxetine, paroxetine, venlafaxine, sertraline, [3,6-Dimethyl-2-(2,4,6-trimethyl-phenoxy)-pyridin-4-yl]-(1-ethylpropyl)-amine and 3,5-dimethyl-4-(3'-pentoxy)-2-(2',4',6'-trimethylphenoxy)pyridine.

40. The dosage form of claim 33 wherein said drug is a glycogen phosphorylase inhibitor selected from the group consisting of [R-(R*S*)]-5-chloro-N-[2-hydroxy-3-[methoxymethylamino}-3-oxo-1-(phenylmethyl)propyl]propyl]-1H-indole-2-carboxamide and 5-chloro-1H-indole-2-carboxylic acid [(1S)-benzl;-3((3R,4S)-dihydroxypyrrolidin-1-yl-)-(2R)-hydroxy-3-oxypropyl]amide.

## Patentansprüche

1. Dosierungsform mit kontrollierter Freigabe, welche das Folgende umfasst:
(a) einen Kern, welcher ein Osmosemittel und ein Medikament in Form einer sprühgetrockneten festen Dispersion des Medikaments in einem Dispersionspolymer umfasst, welches aus Hydroxypropylmethylcellulose, Hydroxypropylmethylcellulosephthalat, Hydroxypropylmethylcelluloseacetatsuccinat, Celluloseacetatphthalat, Celluloseacetattrimellitat und Gemischen davon ausgewählt ist, wobei das Medikament bei einem physiologisch relevanten pH-Wert eine Wasserlöslichkeit von 40 mg/mL oder weniger aufweist und mindestens 60 % des Medikaments amorph sind; und
(b) eine weitgehend wasserdurchlässige Beschichtung um den Kern herum, welche mindestens eine Abgabeöffnung aufweist, wobei die Beschichtung das Eindringen von Wasser aus einer wässrigen Anwendungsumgebung in den Kern kontrolliert, so dass das Ausstoßen mindestens eines Teils des Kerns durch die mindestens eine Abgabeöffnung hindurch in die wässrige Anwendungsumgebung bewirkt wird, wobei sich die Beschichtung während der Freigabe des Medikaments nicht auflöst und nicht abgetragen wird.

2. Dosierungsform nach Anspruch 1, wobei mindestens 75 % des Medikaments amorph sind.

3. Dosierungsform nach Anspruch 1, wobei mindestens 90 % des Medikaments amorph sind.

4. Dosierungsform nach Anspruch 1, wobei es sich bei der Beschichtung um eine Polymermembran handelt.

5. Dosierungsform nach Anspruch 4, wobei die Polymermembran semipermeabel ist.

6. Dosierungsform nach Anspruch 4, wobei die Polymermembran porös ist.

7. Dosierungsform nach Anspruch 4, wobei die Polymermembran mindestens eine asymmetrische Membran umfasst.

8. Dosierungsform nach Anspruch 7, wobei die mindestens eine Abgabeöffnung Poren in der Beschichtung umfasst.

9. Dosierungsform nach Anspruch 1, wobei die mindestens eine Abgabeöffnung durch Laserbohren gebildet wird.

10. Dosierungsform nach Anspruch 1, wobei die mindestens eine Abgabeöffnung in der Anwendungsumgebung gebildet wird.

11. Dosierungsform nach Anspruch 10, wobei mindestens eine Abgabeöffnung durch das Abtragen eines Stopfens aus einem wasserlöslichen Material gebildet wird.

12. Dosierungsform nach Anspruch 10, wobei die Beschichtung aufbrechen kann, um die mindestens eine Abgabeöffnung zu bilden.

13. Dosierungsform nach Anspruch 12, wobei die mindestens eine Abgabeöffnung durch einen Bruch eines relativ kleinen Teils der Beschichtung gebildet wird.

14. Dosierungsform nach Anspruch 13, wobei der Bruch in einem dünneren Teil der Beschichtung über einer Einkerbung in dem Kern stattfindet.

15. Dosierungsform nach Anspruch 4, wobei die Beschichtung aus einem Polymer gebildet wird, das aus der Gruppe ausgewählt ist, die aus Polyacrylsäuren und -estern; Polymethacrylsäuren und -estern; Copolymeren aus Polyacrylsäuren und -estern und Polymethacrylsäuren und - estern; Celluloseestern; Celluloseethern und Celluloseesterethern besteht.

16. Dosierungsform nach Anspruch 4, wobei die Beschichtung aus einem Polymer gebildet wird, das aus der Gruppe ausgewählt ist, die aus Polyethylenglykol, Polypropylenglykol, Copolymeren aus Polyethylenglykol und Polypropylenglykol, Polyvinylpyrrolidon, Ethylcellulose, Hydroxyethylcellulose, Hydroxypropylcellulose, Carboxymethylcellulose, Carboxymethylethylcellulose, Stärke, Dextran, Dextrin, Chitosan, Kollagen, Gelatine, Bromelain, Celluloseacetat, nicht weich gemachtem Celluloseacetat, weich gemachtem Celluloseacetat, verstärktem Celluloseacetat, Celluloseacetatphthalat, Celluloseacetattrimellitat, Hydroxypropylmethylcellulose, Hydroxypropylmethylcellulosephthalat, Hydroxypropylmethylcelluloseacetatsuccinat, Hydroxypropylmethylcelluloseacetattrimellitat, Cellulosenitrat, Cellulosediacetat, Cellulosetriacetat, Agaracetat, Amylosetriacetat, Betaglucanacetat, Betaglucantriacetat, Acetaldehyddimethylacetal, Celluloseacetatethylcarbamat, Celluloseacetatphthalat, Celluloseacetatmethylcarbamat, Celluloseacetatsuccinat, Celluloseacetatdimethylaminoacetat, Celluloseacetatethylcarbonat, Celluloseacetatchloracetat, Celluloseacetatethyloxalat, Celluloseacetatmethylsulfonat, Celluloseacetatbutylsulfonat, Celluloseacetatpropionat, Celluloseacetat-p-toluolsulfonat, Triacetat des Johannisbrotgummis, Celluloseacetat mit acetylierter Hydroxyethylcellulose, hydroxyliertem Ethylen-Vinylacetat, Celluloseacetatbutyrat, Polyalkenen, Polyethern, Polysulfonen, Polyethersulfonen, Polystyrolen, Polyvinylhalogeniden, Polyvinylestern und -ethern, natürlichen Wachsen und synthetischen Wachsen besteht.

17. Dosierungsform nach Anspruch 1 in einer Form, die aus der Gruppe ausgewählt ist, die aus einer Tablette, einer Kapsel, einem Kügelchen und einer Zusammenstellung aus mindestens zwei Arten von Kügelchen mit unterschiedlichen Medikamentfreigabeeigenschaften besteht, und wobei es sich bei der Anwendungsumgebung um einen menschlichen Gastrointestinaltrakt handelt.

18. Dosierungsform nach Anspruch 1, wobei das Osmosemittel aus der Gruppe ausgewählt ist, die aus einem osmotisch wirksamen gelösten Stoff und einem in Wasser quellbaren hydrophilen Polymer besteht.

19. Dosierungsform nach Anspruch 18, wobei das Osmosemittel in Wasser quellbar ist und von der festen Dispersion weitgehend isoliert ist.

20. Dosierungsform nach Anspruch 19, wobei sich das Osmosemittel und die feste Dispersion in entsprechenden unterschiedlichen Schichten befinden.

21. Dosierungsform nach Anspruch 20, wobei sich das Osmosemittel in einer ersten Schicht und die feste Dispersion in einer zweiten Schicht befinden.

22. Dosierungsform nach Anspruch 21, welche die feste Dispersion in einer dritten Schicht umfasst, wobei sich das Osmosemittel zwischen der ersten Schicht und der zweiten Schicht befindet.

23. Dosierungsform nach Anspruch 19, wobei die feste Dispersion das Osmosemittel umgibt.

24. Dosierungsform nach Anspruch 1, wobei es sich bei dem Osmosemittel und dem Dispersionspolymer um denselben Stoff handelt.

25. Dosierungsform nach Anspruch 18, wobei das in Wasser quellbare hydrophile Polymer aus der Gruppe ausgewählt ist, die aus hydrophilen Vinyl- und Acrylpolymeren, Polysacchardidalginaten, Poly(ethylenoxid), Polyethylenglykol, Polypropylenglykol, Poly-(2-hydroxyethylmethycrylat), Polyacrylsäure, Polymethacrylsäure, Polyvinylpyrrolidon, vernetztem Polyvinylpyrrolidon, Polyvinylalkohol, Polyvinylpyrrolidon/PolyvinylalkoholCopolymeren, Polyvinylacetat, hydrophilen Polyurethanen, welche große Poly(ethylenoxid)-Blöcke enthalten, Carrageenan, Hydroxyethylcellulose, Hydroxypropylcellulose, Hydroxypropylmethylcellulose, Carboxymethylcellulose, Carboxyethylcellulose, Natriumalginat, Polycarbophil, Gelatine, Xanthan, Natrium-Croscarmellose und Natrium-Stärkeglycolat besteht.

26. Dosierungsform nach Anspruch 25, wobei das in Wasser quellbare hydrophile Polymer aus der Gruppe ausgewählt ist, die aus Poly(ethylenoxid), Polyethylenglykol, Carboxymethylcellulose, Polyvinylpyrrolidon, Hydroxypropylmethylcellulose, Polyacrylsäure, vernetzter Polyacrylsäure, Natrium-Croscarmellose und Natrium-Stärkeglykolat besteht.

27. Dosierungsform nach Anspruch 1, wobei der Kern ferner einen Löslichkeitsverstärker umfasst.

28. Dosierungsform nach Anspruch 27, wobei der Löslichkeitsverstärker aus der Gruppe ausgewählt ist, die aus organischen Säuren und organischen Säuresalzen; unvollständig veresterten Glyceriden; Glyceriden; Glyceridderivaten; Polyethylenglykolestern; Polypropylenglykolestern; Estern mehrwertiger Alkohole; Polyoxyethylen-Ethern; Sorbitanestern; Polyoxyethylensorbitanestern; Carbonatsalzen und Cyclodextrinen besteht.

29. Dosierungsform nach Anspruch 1, wobei das Medikament vor der Bildung der festen Dispersion amorph ist.

30. Dosierungsform nach Anspruch 1, wobei das Medikament vor der Bildung der festen Dispersion kristallin ist.

31. Dosierungsform nach Anspruch 1, wobei der Kern ferner Zusatzstoffe umfasst.

32. Dosierungsform nach Anspruch 31, wobei die Zusatzstoffe aus der Gruppe ausgewählt sind, die aus oberflächenaktiven Stoffen, wasserlöslichen Polymeren, pH-Wert-Modifikatoren, Füllstoffen, Bindemitteln, Pigmenten, Gleitmitteln, Antioxidationsmitteln und Geschmacksstoffen besteht.

33. Dosierungsform nach Anspruch 1, wobei das Medikament aus der Gruppe ausgewählt ist, die aus einem Antihypertonikum, einem angstlösenden Mittel, einem Mittel gegen Blutgerinnsel, einem Blutzucker-Senkungsmittel, einem Entstauungsmittel, einem Antihistamin, einem Hustenmittel, einem Entzündungshemmer, einem Anti-Atherosklerose-Mittel, einem Antipsychotikum, einem Nootropikum, einem Cholesterinsenker, einem Appetitzügler, einem Mittel gegen Autoimmunkrankheiten, einem Hypnotikum, einem Parkinson-Mittel, einem Antibiotikum, einem Antivirusmittel, einem Potenzmittel, einem Antineoplastikum, einem Sedativum, einem Barbiturat, einem Nahrungsmittel, einem Betablocker, einem Brechmittel, einem Mittel gegen Erbrechen, einem Diuretikum, einem Gerinnungshemmer, einem Kardiotonikum, einem Androgen, einem Corticoid, einem Anabolikum, einem Antidepressivum, einem Antiinfektionsmittel, einem koronaren Vasodilator, einem Carboanhydrasehemmer, einem Antimykotikum, einem Antiprotozoenmittel, einem Magen-Darm-Mittel, einem dopaminergen Mittel, einem Alzheimer-Mittel, einem Antiulzerativum, einem Thrombozytenhemmer und einem Glycogenphosphorylase-Hemmer besteht.

34. Dosierungsform nach Anspruch 33, wobei es sich bei dem Medikament um ein Antihypertonikum handelt, das aus der Gruppe ausgewählt ist, die aus Prazosin, Nifedipin, Trimazosin und Doxazosin besteht.

35. Dosierungsform nach Anspruch 33, wobei es sich bei dem Medikament um das Antipsychotikum Ziprasidon handelt.

36. Dosierungsform nach Anspruch 33, wobei es sich bei dem Medikament um das Blutzucker-Senkungsmittel Glipizid handelt.

37. Dosierungsform nach Anspruch 33, wobei es sich bei dem Medikament um ein Potenzmittel handelt, das aus der Gruppe ausgewählt ist, die aus Sildenafil und dessen pharmazeutisch unbedenklichen Salzen besteht.

38. Dosierungsform nach Anspruch 33, wobei es sich bei dem Medikament um den Entzündungshemmer (+)-N-{4-[3-(4-Fluorphenoxy)phenoxy]-2-cyclopenten-1-yl}-N-hydroxyharnstoff handelt.

39. Dosierungsform nach Anspruch 33, wobei es sich bei dem Medikament um ein Antidepressivum handelt, das aus der Gruppe ausgewählt ist, die aus Fluoxetin, Paroxetin, Venlafaxin, Sertralin, [3,6-Dimethyl-2-(2,4,6-trimethylphenoxy)pyridin-4-yl]-(1-ethylpropyl)amin und 3,5-Dimethyl-4-(3'-pentoxy)-2-(2',4',6'-trimethylphenoxy)pyridin besteht.

40. Dosierungsform nach Anspruch 33, wobei es sich bei dem Medikament um einen Glycogenphosphorylase-Hemmer handelt, der aus der Gruppe ausgewählt ist, die aus [R-(R*S*)]-5-Chlor-N-[2-hydroxy-3-[methoxymethylamino-3-oxo-1-(phenylmethyl)propyl]propyl]-1H-indol-2-carboxamid und 5-Chlor-1H-indol-2-carbonsäure-[(1S)-benzyl-3-((3R,4S)-dihydroxypyrrolidin-1-yl)-(2R)-hydroxy-3-oxypropyl]amid besteht.

## Revendications

1. Forme pharmaceutique à libération contrôlée, comprenant :
(a) un noyau comprenant un agent osmotique et un médicament sous la forme d'une dispersion de solides, séchée par atomisation, du dit médicament dans un polymère de dispersion choisi parmi l'hydroxypropylméthylcellulose, le phtalate d'hydroxypropylméthylcellulose, l'acétate succinate d'hydroxypropylméthylcellulose, l'acétate phtalate de cellulose, l'acétate trimellitate de cellulose et leurs mélanges, dans laquelle le médicament a une solubilité en milieu aqueux de 40 mg/ml ou moins à un pH physiologiquement approprié, au moins 60 % du dit médicament étant amorphes ; et
(b) un enrobage à peu près perméable à l'eau autour dudit noyau, comportant au moins un orifice d'évacuation, ledit enrobage contrôlant l'afflux d'eau dans ledit noyau à partir d'un environnement d'utilisation aqueux, de façon à provoquer l'expulsion d'au moins une partie du dit noyau à travers ledit au moins un orifice d'évacuation dans ledit environnement aqueux d'utilisation, ledit enrobage étant non soluble et non érodable au cours de la libération du dit médicament.

2. La forme pharmaceutique de la revendication 1, dans laquelle au moins 75 % du dit médicament sont amorphes.

3. La forme pharmaceutique de la revendication 1, dans laquelle au moins 90 % du dit médicament sont amorphes.

4. La forme pharmaceutique de la revendication 1, dans laquelle ledit enrobage est une membrane de polymère.

5. La forme pharmaceutique de la revendication 4, dans laquelle ladite membrane de polymère est semiperméable.

6. La forme pharmaceutique de la revendication 4, dans laquelle ladite membrane de polymère est poreuse.

7. La forme pharmaceutique de la revendication 4, dans laquelle ladite membrane de polymère comprend au moins une membrane asymétrique.

8. La forme pharmaceutique de la revendication 7, dans laquelle ledit au moins un orifice d'évacuation comprend des pores dans ledit enrobage.

9. La forme pharmaceutique de la revendication 1, dans laquelle ledit au moins un orifice d'évacuation est formé par forage au laser.

10. La forme pharmaceutique de la revendication 1, dans laquelle ledit au moins un orifice d'évacuation est formé dans ledit environnement d'utilisation.

11. La forme pharmaceutique de la revendication 10, dans laquelle le dit au moins un orifice d'évacuation est formé par l'érosion d'un bouchon d'une matière soluble dans l'eau.

12. La forme pharmaceutique de la revendication 10, dans laquelle ledit enrobage peut être rompu pour former ledit au moins un orifice d'évacuation.

13. La forme pharmaceutique de la revendication 12, dans laquelle ledit au moins un orifice d'évacuation est formé par rupture d'une relativement petite partie dudit enrobage.

14. La forme pharmaceutique de la revendication 13, dans laquelle ladite rupture a lieu dans une partie relativement mince dudit enrobage par-une indentation dans le dit noyau.

15. La forme pharmaceutique de la revendication 4, dans laquelle ledit enrobage est formé à partir d'un polymère choisi parmi le groupe consistant en les acides et les esters poly(acryliques) ; les copolymères des acides et des esters poly(acryliques) et poly(méthacryliques) ; les esters de cellulose ; les éthers de cellulose ; et les esters/éthers de cellulose.

16. La forme pharmaceutique de la revendication 4, dans laquelle ledit enrobage est formé à partir d'un polymère choisi parmi le groupe consistant en le polyéthylène glycol, le polypropylène glycol, les copolymères de polyéthylène glycol et de polypropylène glycol, la poly (vinylpyrrolidone), l'éthyl cellulose, l'hydroxyéthyl cellulose, l'hydroxypropyl cellulose, le carboxyméthyl cellulose, le carboxyméthyléthyl cellulose, l'amidon, le dextrane, la dextrine, le chitosane, le collagène, la gélatine, la bromélaïne, l'acétate de cellulose, l'acétate de cellulose non plastifié, l'acétate de cellulose plastifié, l'acétate de cellulose renforcé, l'acétate phtalate de cellulose, l'acétate trimellitate de cellulose, l'hydroxypropylméthylcellulose, le phtalate d'hydroxypropylméthylcellulose, l'acétate succinate d'hydroxypropylméthylcellulose, l'acétate trimellitate d'hydroxypropylméthylcellulose, le nitrate de cellulose, le diacétate de cellulose, le triacétate de cellulose, l'acétate de gélose, le triacétate d'amylose, l'acétate de bêta glucane, le triacétate de bêta glucane, le diméthyl acétate d'acétaldéhyde, l'éthyl carbamate acétate de cellulose, l'acétate phtalate de cellulose, l'acétate méthyl carbamate de cellulose, acétate succinate de cellulose, l'acétate diméthaminoacétate de cellulose, l'acétate éthyl carbonate de cellulose, l'acétate chloroacétate de cellulose, l'acétate éthyl oxalate de cellulose, l'acétate méthyl sulfonate de cellulose, l'acétate butyl sulfonate de cellulose, l'acétate proprionate de cellulose, l'acétate p-toluène sulfonate de cellulose, le triacétate de gomme de caroube, l'acétate de cellulose avec de l'hydroxyéthyl cellulose acétylée, l'éthylène acétate de vinyle hydroxylé, l'acétate butyrate de cellulose, les polyalcènes, les polyéthers, les polysulfones, les polyéthersulfones, les polystyrènes, les polyhalogénures de vinyle, les polyesters et les polyéthers de vinyle, les cires naturelles et les cires synthétiques.

17. La forme pharmaceutique de la revendication 1, sous une forme choisie parmi le groupe consistant en un comprimé, une capsule, un grain et une collection d'au moins deux types de grains ayant des propriétés de libération de médicament différentes, et dans laquelle l'environnement d'utilisation est un tube digestif humain.

18. La forme pharmaceutique de la revendication 1, dans laquelle ledit agent osmotique est choisi parmi le groupe consistant en un soluté à effet osmotique et un polymère hydrophile gonflable à l'eau.

19. La forme pharmaceutique de la revendication 18, dans laquelle ledit agent osmotique est gonflable à l'eau, et à peu près séparé de ladite dispersion de solides.

20. La forme pharmaceutique de la revendication 19, dans laquelle ledit agent osmotique et ladite dispersion de solides sont dans des couches discrètes respectives.

21. La forme pharmaceutique de la revendication 20, dans laquelle ledit agent osmotique est dans une première couche, et ladite dispersion de solides est dans une deuxième couche.

22. La forme pharmaceutique de la revendication 21, contenant une dispersion de solides dans une troisième couche, ledit agent osmotique étant entre ladite première couche et ladite deuxième couche.

23. La forme pharmaceutique de la revendication 19, dans laquelle ladite dispersion de solides entoure ledit agent osmotique.

24. La forme pharmaceutique de la revendication 1, dans laquelle ledit agent osmotique et ledit polymère de dispersion sont les mêmes.

25. La forme pharmaceutique de la revendication 18, dans laquelle ledit polymère hydrophile gonflable à l'eau est choisi parmi le groupe consistant en les polymères vinyliques et acryliques hydrophiles, les polysaccharide alginates, le polyoxyde d'éthylène, le polyéthylène glycol, le polypropylène glycol, le polyméthacrylate de 2-hydroxyéthyle, l'acide polyacrylique, l'acide polyméthacrylique, la polyvinyl pyrrolidone, la polyvinyl pyrrolidone réticulée, l'alcool polyvinylique, les copolymères de polyvinyl pyrrolidone et d'alcool polyvinylique, l'acétate de vinyle, les polyuréthanes hydrophiles contenant de grands segments de polyoxyde d'éthylène, la carragénine, l'hydroxyéthylcellulose, l'hydroxypropylcellulose, l'hydroxypropylméthylcellulose, la carboxyméthylcellulose, la carboxyléthylcellulose, l'alginate de sodium, le polycarbophile, la gélatine, la gamme de xanthane, la crosscarmellose sodique et l'amidon glycolate de sodium.

26. La forme pharmaceutique de la revendication 25, dans laquelle ledit polymère hydrophile est choisi parmi le groupe consistant en le polyoxyde d'éthylène, le polyéthylène glycol, la carboxyméthylcellulose, la polyvinylpyrrolidone, l'hydroxypropylméthylcellulose, l'acide polyacrylique, l'acide polyacrylique réticulé, la crosscarmellose sodique et l'amidon glycolate de sodium.

27. La forme pharmaceutique de la revendication 1, dans laquelle ledit noyau comprend, en outre, un agent d'accroissement de solubilité.

28. La forme pharmaceutique de la revendication 27, dans laquelle ledit agent d'accroissement de solubilité est choisi parmi le groupe consistant en les acides organiques et les sels d'acide organique ; les glycérides partiels ; les glycérides ; les dérivés de glycéride ; les esters de polyéthylène glycol ; les esters de polypropylène glycol ; les esters de polyol ; les éthers de polyoxyéthylène ; les esters de sorbitanne ; les esters de polyoxyéthylène sorbitanne ; les sels de type carbonate ; et les cyclodextrines.

29. La forme pharmaceutique de la revendication 1, dans laquelle, avant la formation de ladite dispersion de solides, ledit médicament est amorphe.

30. La forme pharmaceutique de la revendication 1, dans laquelle, avant la formation de ladite dispersion de solides, ledit médicament est cristallin.

31. La forme pharmaceutique de la revendication 1, dans laquelle ledit noyau comprend, en outre, des excipients.

32. La forme pharmaceutique de la revendication 31, dans laquelle lesdits excipients sont choisis parmi le groupe consistant en les tensio-actifs, les polymères solubles dans l'eau, les modificateurs de pH, les charges, les liants, les pigments, les lubrifiants, les antioxydants et les agents aromatisants.

33. La forme pharmaceutique de la revendication 1, dans laquelle ledit médicament est choisi parmi le groupe consistant en un agent antihypertenseur, et un agent anxiolytique, un agent anticoagulant, un agent de diminution du glucose sanguin, un agent décongestionnant, un agent antihistaminique, un agent antitussif, un anti-inflammatoire, un agent antiathéroscléreux, un agent anti-psychotique, un agent d'accroissement de la cognition, un agent de diminution du cholestérol, un agent antiobésité, un agent contre les troubles autoimmuns, un agent hypnotique, un agent antiparkinsonien, un antibiotique, un agent antiviral, un agent contre l'impuissance, un agent antinéoplasique, un sédatif, un barbiturique, un agent nutritionnel, un bêta bloquant, un émétique, un antiémétique, un diurétique, un anticoagulant, un cardiotonique, un androgène, un corticoïde, un anabolisant, un antidépresseur, un anti-infectieux, un vasodilatateur coronaire, un inhibiteur d'anhydrase carbonique, un agent antifongique, un agent antiprotozoaire, un agent gastro-intestinal, un agent dopaminergique, un agent contre la maladie d'Alzheimer, un agent antiulcéreux, un inhibiteur de plaquettes, et un inhibiteur de glycogène phosphorylase.

34. La forme pharmaceutique de la revendication 33, dans laquelle ledit médicament est un agent antihypertenseur choisi parmi le groupe consistant en la prazosine, la nifédipine, la trimazosine et la doxazosine.

35. La forme pharmaceutique de la revendication 33, dans laquelle ledit médicament est l'agent antipsychotique ziprasidone.

36. La forme pharmaceutique de la revendication 33, dans laquelle ledit médicament est l'agent de diminution du glucose sanguin glipizide.

37. La forme pharmaceutique de la revendication 33, dans laquelle ledit médicament est un agent contre l'impuissance choisi parmi le groupe consistant en le sildénafil et les sels pharmaceutiquement acceptables de celui-ci.

38. La forme pharmaceutique de la revendication 33, dans laquelle ledit médicament est l'agent anti-inflammatoire (+)-N-{4-[3-(4-fluorophénoxy) phénoxy]-2-cyclopentén-1-yl}-N-hydroxyurée.

39. La forme pharmaceutique de la revendication 33, dans laquelle ledit médicament est un agent antidépresseur choisi parmi le groupe consistant en la fluoxétine, la paroxétine, la venlafaxine, la sertraline, la [3,6-diméthyl-2-(2,4,6 triméthyl-phénoxy)-pyridin-4-yl]-(1-éthylpropyl)-amine et la 3,5-diméthyl-4-(3'-pentoxy)-2-(2',4',6'-triméthylphénoxy)pyridine.

40. La forme pharmaceutique de la revendication 33, dans laquelle ledit médicament est un inhibiteur de glycogène phosphorylase choisi parmi le groupe consistant en le [R-(R'S')]-5-chloro-N-[2-hydroxy-3-[méthoxyméthylamino]-3-oxo-1-(phénylméthyl)propyl] propyl]-1H-indole-2-carboxamide et le [(1S)-benzyl-3 ((3R,4S)-dihydroxypyrrolidin-1-yl)-(2R)-hydroxy-3-oxypropyl]amide de l'acide 5-chloro-1H-indole-2-carboxylique.
